# EUROPEAN PATENT APPLICATION

(11) **EP 2 388 270 A2**
(43) Date of publication of application: **23.11.2011**
(21) Application number: 11169933.6
(22) Date of filing: 10.10.2008
(51) Int. Cl.: C07K 14/705, A61K 38/17, A61K 39/395, A61K 47/48, C07K 16/28, C07K 19/00, C12N 15/12, C12Q 1/68

(54) **Modulation of SIRPa - CD47 interaction for increasing human hematopoietic stem cell engraftment and compounds therefor**

(30) Priority: 11.10.2007 US 960724 P
(62) Divisional of application: 08838285.8
(71) Applicant: The Hospital For Sick Children, Toronto, Ontario M5G 1X8 (CA); University Health Network, Toronto, ON M5G 2C4 (CA)
(72) Inventor: Danska, Jane, Toronto M5R 3C4 (CA); Dick, John, Ontario, M4E 2Z8 (CA); Prasolava, Tatiana, Ontario M5H 4E9 (CA); Takenaka, Katsuto, Fukuoka, 819-0005 (JP)
(74) Representative: Cabinet Plasseraud

(57) **Abstract**

The invention relates to modulating the SIRPα - CD47 interaction in order to increase hematopoietic stem cell engraftment and compounds therefor. In some embodiments, there is provided isolated SIRPα and CD47 polypeptides, fragments and fusion proteins for enhancing hematopoietic stem cell engraftment. Further there is provided methods for increasing hematopoietic stem cell engraftment through administration of the above polypeptides.

## Description

### FIELD OF THE INVENTION

The invention relates to modulating the SIRPα - CD47 interaction in order to increase hematopoietic stem cell engraftment and compounds therefor. In some embodiments, there is provided isolated SIRPα and CD47 polypeptides, fragments and fusion proteins for enhancing hematopoietic stem cell engraftment. Further there is provided methods for increasing hematopoietic stem cell engraftment through administration of the above polypeptides.

### BACKGROUND OF THE INVENTION

The transplantation of human hematopoietic stem cells (HSC) from bone marrow (BM) or G-CSF mobilized peripheral blood (PB) has been one of the most important clinical applications of stem cell biology. HSC transplantation in individuals with neoplastic disease enables the use of a high dose chemotherapy regimen and subsequent HSC rescue to overcome the resultant hematopoietic failure due to chemotherapy, enhancing cure rates for both hematologic and non-hematologic tumors. Additionally, genetic diseases such as thalassemia and certain immune-deficiencies can be managed by autologous transplantation of gene-corrected HSC or by transplantation of allogeneic HSC. A key discovery enabling successful HSC transplantation was identification of the human leukocyte antigen (HLA) system as the human major histocompatibility complex. Although HLA disparity between donor and host plays a major role in graft rejection, graft failure can occur even in patients receiving an unmanipulated, HLA-identical transplant (Thomas, E.D. et al. (1977) Blood 49, 511-33; Storb, R. et al. (1977) N Engl J Med 296, 61-6). Graft rejection in this setting may be related in part to mismatch at minor histocompatibility antigens (Gale, R.P. et al. (1981) Blood 57, 9-12). Additional genes other than the HLA haplotypes that modulate HSC engraftment have not been characterized.

HSC reside in supportive microenvironmental niches comprised of fibroblastic stroma, osteoblasts, osteoclasts, macrophages, and endothelial cells (Suda, T. et al. (2005) Trends Immunol 26, 426-33). Abrogation of HSC interaction with such niches, for example by blocking specific adhesion proteins or chemokine receptors, prevents HSC engraftment (Lapidot, T. et al. (2005) Blood 106, 1901-10). Additionally, natural killer (NK) cells and macrophages, both components of the innate immune system, have been shown to play a role in murine HSC transplantation (Murphy, W.J. et al. (1987) J Exp Med 165, 1212-7) and human hematopoietic xenotransplantation (McKenzie, J.L. et al. (2005) Blood 106, 1259-61), respectively. A better understanding of the mechanisms underlying hematopoietic stem cell engraftment following transplantation, including genes that control regulatory pathways, could ultimately translate into better clinical outcomes.

Xenotransplantation in the non-obese diabetic/severe combine immune-deficient NOD*.Pdck*^{*sc*/*sc*} (NOD.*SCID*) mouse has become the "gold standard" assay for human HSC engraftment. The assay is based on the ability of human HSC to repopulate the immune system of these animals following intravenous injection (Wang, J.C.Y. et al. "Normal and leukemic human stem cells assayed in immune-deficient mice" in: Hematopoiesis - A Developmental Approach (ed. Zon, L.I.) 99-118 (Oxford University Press, New York, USA, 2001). The cells that initiate the human xenograft are operationally defined as SCID-repopulating cells (SRC), possess properties attributed to HSC, and are distinct from more mature progenitors assayed *in vitro.* This system has enabled analysis of the proliferation, differentiation, and self renewal properties of SRC within the human HSC compartment.

Signal regulatory proteins (SIRPs) constitute a family of cell surface glycoproteins which are expressed on myeloid (including macrophages, granulocytes, myeloid dendritic cells, and mast cells) and neuronal cells (summarized in Barclay, A.N. & Brown, M.H., Nat Rev Immunol 6, 457-64 (2006); see also WO 97/48723). SIRPs constitute a diverse multigene family of immune receptors encompassing inhibitory (SIRPα), activating (SIRPβ), nonsignaling (SIRPγ) and soluble (SIRPδ) members. CD47, a broadly expressed transmembrane glycoprotein, functions as a cellular ligand for SIRPα and binds to the NH₂-terminal extracellular terminus of SIRPα. SIRPα's role has been best documented in respect of its inhibitory role in the phagocytosis of host cells by macrophages. In particular, the binding of SIRPα on macrophages by CD47 expressed on target cells, generates an inhibitory signal that negatively regulates phagocytosis. However, more recent findings have also demonstrated additional positive signaling effects mediated through SIRPα binding (Shultz, L.D. et al. (1995) J Immunol 154, 180-91).

A variety of approaches have been proposed to disrupt the CD47- SIRPα interaction in an effort to effect a biological outcome. These encompass the use of fragmented/truncated SIRPα and/or CD47 proteins and antibodies thereto (see, for example, International Patent Application Publication No. WO 99/40940; U.S. Patent Application Publication Nos. 2003/0026803 and 2006/0135749; and U.S. Patent No. 6,913,894), for modifying immune function (see, for example, International Patent Application Publication No. WO 99/40940; and U.S. Patent Application Publication Nos. 2003/0026803 and 2007/0113297), and for xenotransplantation (see, for example, U.S. Patent Application Publication Nos. 2005/0255550, and 2007/0157328).

There remains a need to identify molecules capable of supporting HSC engraftment and **hematopoiesis upon transplantation.**

### SUMMARY OF THE INVENTION

According to one aspect, there is provided an isolated polypeptide selected from the group consisting of a polypeptide consisting of a) the amino acid sequence of SEQ ID NO. 1; b) a polypeptide consisting of a fragment of the amino acid sequence of SEQ ID NO. 1, wherein the fragment comprises at least one of residues 31, 32, 34, 37, 74, 77, 83, 84, 86, 87, 90, 91, 96, 100, 102, 114, 118, 126 of SEQ ID NO. 1; and c) one of the polypeptide in a) and b) with up to 1 amino acid insertion, deletion or substitution for every 7 amino acids in length of the polypeptide, wherein the polypeptide comprises at least one of residues 31, 32, 34, 37, 74, 77, 83, 84, 86, 87, 90, 91, 96, 100, 102, 114, 118, 126 of SEQ ID NO. 1 and binds human CD47.

According to a further aspect, there is provided an isolated polypeptide selected from the group consisting of a) a polypeptide consisting of the amino acid sequence of SEQ ID NO. 2; b) a polypeptide consisting of a fragment of the amino acid sequence of SEQ ID NO. 2; and c) one of the polypeptide in a) and b) with up to 1 amino acid insertion, deletion or substitution for every 7 amino acids in length of the polypeptide; wherein i) at least one of residues at positions 31, 32, 34, 37, 74, 77, 83, 84, 86, 87, 90, 91, 96, 100, 102, 114, 118, 126 of SEQ ID NO. 2 in the polypeptide is replaced with corresponding residues 31, 32, 34, 37, 74, 77, 83, 84, 86, 87, 90, 91, 96, 100, 102, 114, 118, 126 of SEQ ID NO. 1; or ii) at least one of residues 129 and 130 of SEQ ID NO. 2 in the polypeptide is deleted.

According to a further aspect, there is provided an isolated polypeptide selected from the group consisting of a) a polypeptide consisting of an amino acid sequence selected from the group consisting of SEQ ID NOS. 4-13; b) a polypeptide consisting of a fragment of an amino acid sequence selected from the group consisting of SEQ ID NOS. 4-13; and c) one of the polypeptide in a) and b) with up to 1 amino acid insertion, deletion or substitution for every 7 amino acids in length of the polypeptide, wherein the polypeptide binds human CD47.

According to a further aspect there is provided a polypeptide capable of binding to human Sirpα; and antibodies to human Sirpα. Preferably, the polypeptide is the extracellular domain of human CD47 fused to the Fc portion of IgG.

According to a further aspect, there is provided a pharmaceutical composition comprising a polypeptide described herein and a pharmaceutically acceptable carrier.

According to a further aspect, there is provided a method for increasing hematopoietic stem cell engraftment in a mammal comprising administering to the mammal a therapeutically effective amount of a polypeptide described herein.

According to a further aspect, there is provided a use of a polypeptide described herein for increasing hematopoietic stem cell engraftment in a mammal or in the preparation of a medicament for increasing hematopoietic stem cell engraftment in a mammal.

According to a further aspect, there is provided a polypeptide described herein for increasing hematopoietic stem cell engraftment in a mammal.

According to a further aspect, there is provided an isolated nucleic acid comprising a sequence that encodes a polypeptide described herein.

According to a further aspect, there is provided an expression vector comprising a nucleic acid described herein.

According to a further aspect, there is provided a cultured cell comprising a vector described herein.

According to a further aspect, there is provided a method of producing a polypeptide comprising culturing a cell described herein under conditions permitting expression of the polypeptide.

According to a further aspect, there is provided a method for increasing hematopoietic stem cell engraftment in a human comprising modulating the interaction between human Sirpα and human CD47.

According to a further aspect, there is provided a method of identifying a compound that increases hematopoietic stem cell engraftment in a human comprising a) contacting at least one of the extracellular domain of human Sirpα and human CD47 with at least one test compound; b) determining the at least one test compound as binding to the at least one of human Sirpα and human CD47; c) contacting the test compound with human hematopoietic cells in a stromal environment; and d) determining whether hematopoietic stem cell engraftment is increased in the presence of the test compound.

According to a further aspect, there is provided a method of determining genetic polymorphisms in humans affecting hematopoietic stem cell engraftment comprising a) sequencing the Sirpα gene from a plurality of humans having undergone hematopoietic transplantation; b) determining nucleotide differences in the Sirpα gene within the plurality of humans; and c) correlating the nucleotide differences with hematopoietic stem cell engraftment to determine relevant polymorphisms.

According to a further aspect, there is provided a method of determining likelihood of hematopoietic stem cell engraftment in a recipient comprising a) sequencing the Sirpα gene from the recipient; and b) determining whether the relevant polymorphisms exist.

### BRIEF DESCRIPTION OF FIGURES

These and other features of the preferred embodiments of the invention will become more apparent in the following detailed description in which reference is made to the appended drawings wherein:

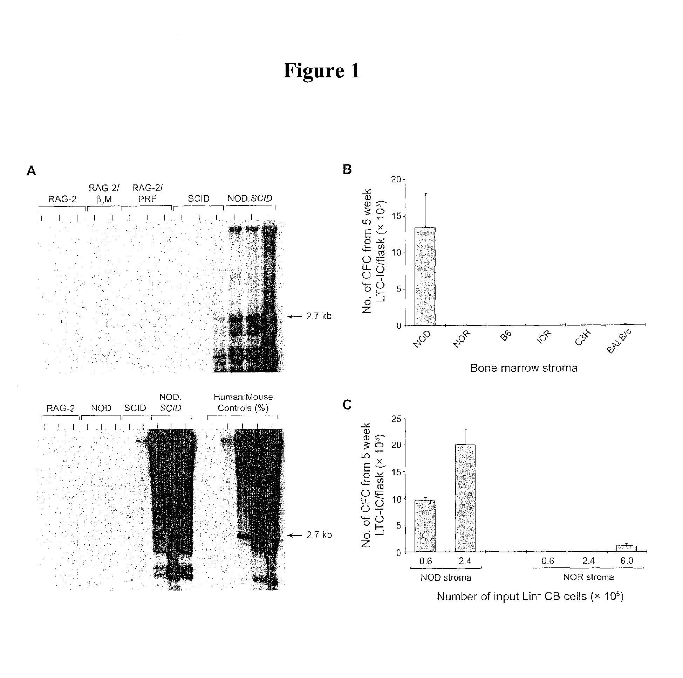

**Figure 1** illustrates the support of human hematopoiesis *in vivo* and *in vitro* in the NOD strain background. (a) Southern blot analysis of DNA extracted from the BM of mice four weeks post-transplant with human BM (top panel; cell dose: RAG-2 knockout strains 40 × 10⁶ mononuclear cells, SCID and NOD/SCID mice 20 × 10⁶ cells) or CB (bottom panel, 15 × 10⁶ mononuclear cells per recipient) cells. The level of human cell engraftment was determined by comparing the intensity of the 2.7 kb α-satellite band (arrow) in the sample lanes with the control human/mouse DNA mixtures. (b, c) Number of CFC generated in chimeric LTC of human Lin⁻ CB cells on irradiated mouse BM stroma. BM cells from NOD, NOR, B6, ICR, C3H, and BALB/c male mice as indicated were cultured for 5 weeks followed by irradiation and subsequent inoculation with 1.1 × 10⁵ (b) or 0.6 - 6.0 × 10⁵ (c) Lin⁻ CB cells. After 5 weeks of culture, cells were harvested and the total number of CFC was determined in standard clonogenic methylcellulose assays. The data is shown as the mean of 3 to 6 independent experiments.

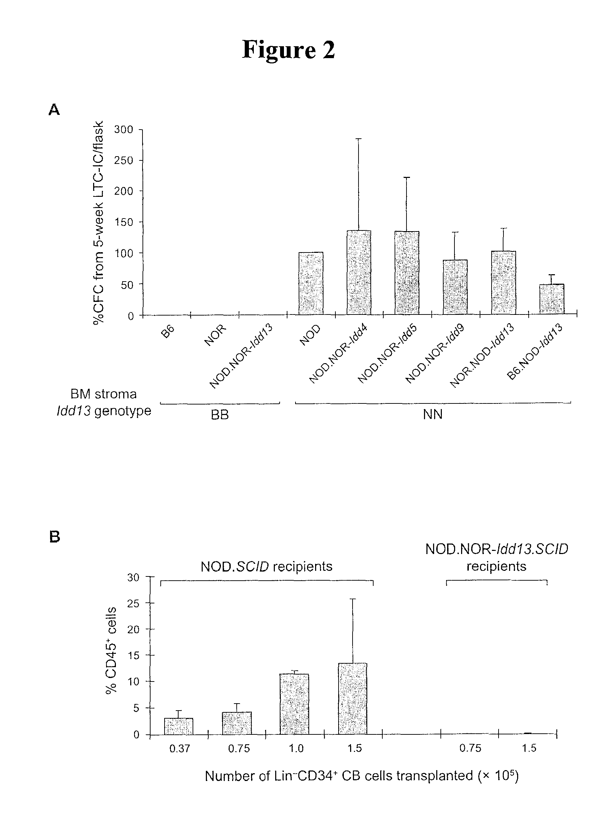

**Figure 2** illustrates the effects of strain-specific differences at the *Idd13* locus on support of human hematopoietic engraftment *in vitro* and *in vivo.* (a) Generation of CFC in chimeric LTC of human Lin⁻ CB cells on mouse stroma from NOD congenic (NOD.NOR-*Idd4*, NOD.NOR-*Idd5*, NOD.NOR-*Idd9*, NOD.NOR-*Idd13*), NOR congenic (NOR.NOD-*Idd13*), and B6.NOD-*Idd13* mice. Murine BM stromal layers were established as described in Fig. 1b and seeded with 0.36 - 2.0 × 10⁵ human Lin⁻ CB cells. After 5 weeks of culture, cells were harvested and the number of CFC was determined by plating in methylcellulose assays. In each experiment, NOD stroma was used as a positive control, and B6 and NOR stromal cells were used as negative controls. The data is normalized to the number of CFC generated in NOD cultures (=100%), and is shown as the mean of at least 3 independent cultures. BB, *Idd13* homozygous B6 BM stroma; NN, *Idd13* genotype homozygous NOD BM stroma. (b) Eight- to 9-week-old NOD*.SCID* (n=11) or NOD.NOR*-Idd13.SCID* (n=8) mice were irradiated with 350 cGy and injected intravenously with 0.37 -1.5 × 10⁵ Lin⁻CD34⁺ cells). Six to 7 weeks post-transplantation, mice were sacrificed and the recipients' BM was assessed for human CD45⁺ cell engraftment by flow cytometry.

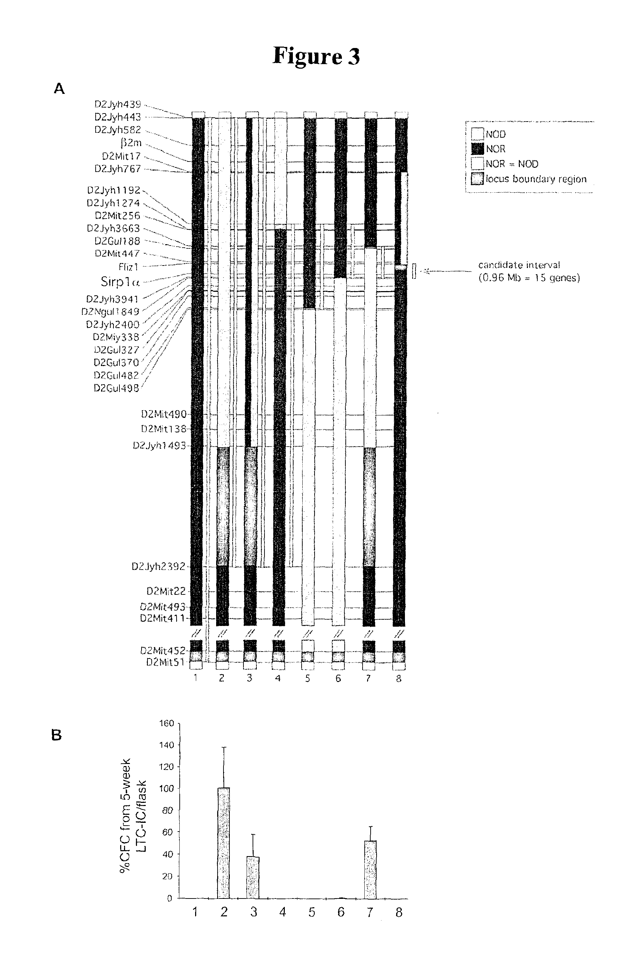

**Figure 3** illustrates the identification of the critical region of *Idd13* associated with support of human hematopoiesis. (a) Graphical representation of chromosome 2 in various congenic strains on both the NOD and NOR backgrounds. Genetic mapping of the candidate interval is denoted by the striped bars to the right of the chromosomal representations. Formal congenic strain designations are as follows: 1. NOD.NOR-D2Jyh443-D2Mit452, 2. NOR.NOD-D2Jyh443-D2Jyh1493, 3. NOR.BN-D2Jyh443-D2Jyh1493, 4. NOR.NOD-D1Ngul46-Ramp1/D2Jyh443-D2Jyh1192, 5. NOD.NOR-D2Jyh443-D2Gul482, 6. NOD.NOR-D2Jyh443-D2Jyh3941, 7. NOR.NOD-DlNgul46-Ramp1/D2Gul188-D2Jyh1493, 8. NOR.BN-D2Jyh767-Flizl. (b) Generation of CFC in chimeric LTC of human Lin⁻ CB cells on mouse stroma from NOD or NOR *Idd13* subcongenic mice depicted in a. Cultures performed and data expressed as in Fig. 2a. Strain designations as described in a.
**Figure 4** illustrates protein sequence alignment of SIRPα. cDNA prepared from BM-derived macrophages was used as a template for PCR amplification of *Sirp*α transcripts from NOD and NOR mice. The B6 mouse sequence is from the EnsEMBL database. Protein domains are indicated by open boxes.

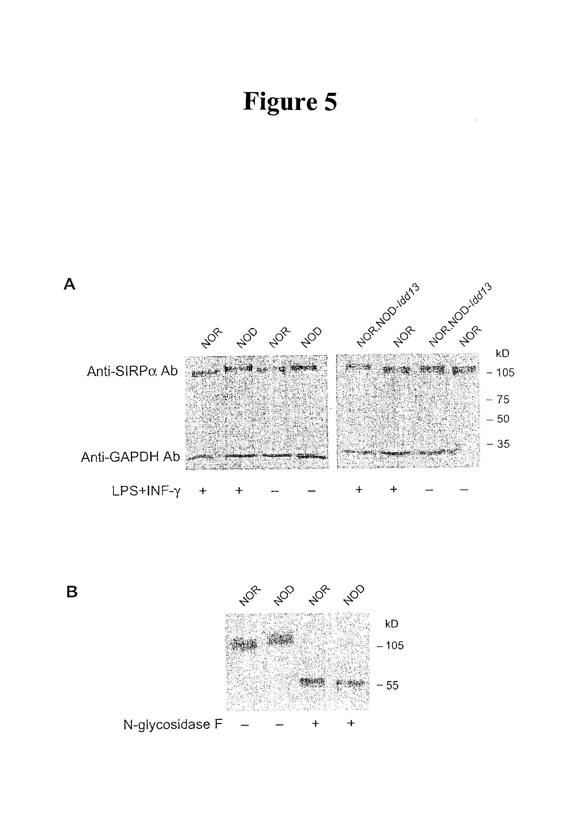

**Figure 5** illustrates immunoblot analysis of SIRPα in BM-derived macrophages from NOD, NOR, and congenic NOR.NOD-*Idd13* mouse strains. (a) Lysates prepared from BM-derived macrophages, either untreated or treated with LPS (100ng/ml) and IFN-γ (10ng/ml), were subjected to immunoblot analysis using polyclonal antibodies directed against the cytoplasmic domain of SIRPα, or glyceraldehyde-3-phosphate dehydrogenase (GAPDH) as a loading control. (b) Lysates prepared from BM-derived macrophages were incubated in the absence (-) or presence (+) of N-glycosidase F for 12 hours at 37°C and then subjected to immunoblot analysis using polyclonal antibodies directed against the cytoplasmic domain of SIRPα. N-glycosidase F treated and untreated samples were co-electrophoresed. Molecular size standards are indicated in kilo Daltons (kD).

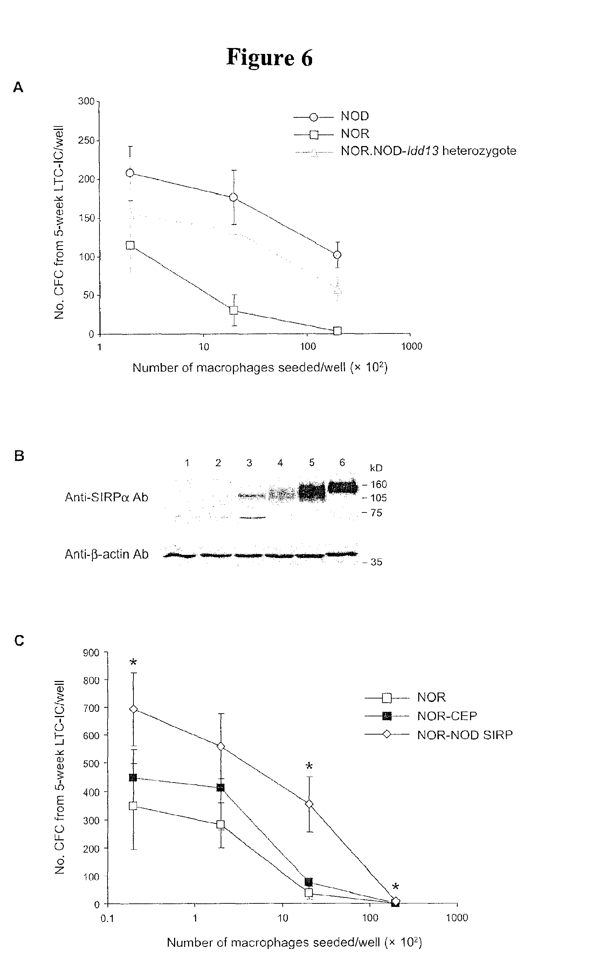

**Figure 6** illustrates *Sirp*α modulation of murine macrophage-mediated suppression of human hematopoiesis. (a) Macrophage-mediated suppression of human hematopoiesis in LTC. MS-5 cells were seeded into 96-well tissue culture plates (2000 cells/well). Peritoneal macrophages harvested from NOD, NOR, and NOR.NOD-*Idd13* heterozygous mice were seeded at doses of 200-20,000 cells/well (5 replicates per dose) with addition of 2000 human Lin⁻ CB cells per well the next day. After 3-4 weeks of culture, cells were harvested and the number of human CFC was determined by plating in methylcellulose assays. Error bars represent standard deviations. (b) Western blot analysis showing SIRPα expression in Jurkat cells and NOR BM-derived macrophages before and after infection with control virus (CEP) or virus expressing NOD-derived *Sirp*α (SIRP). Protein lysates were immunoblotted with polyclonal antibody directed against SIRPα. Lane 1: Jurkat cells; lane 2: Jurkat cells infected with CEP; lane 3: Jurkat cells infected with SIRP; lane 4: NOR macrophages; lane 5: NOR macrophages infected with SIRP; lane 6: NOD macrophages. Infection with SIRP lentivirus resulted in high level expression of NOD SIRPα in NOR macrophages (lane 5). (c) Effect of *Sirp*α on macrophage-mediated suppression of human hematopoiesis. NOR BM-derived macrophages were infected with control (NOR-CEP) lentivirus or lentivirus expressing the NOD-derived *Sirp*α gene (NOR-NOD SIRP) prior to seeding onto established MS-5 stromal cultures at doses of 20 to 20,000 cells/well (5 replicates per dose). 2000 Lin⁻ CB cells/well were added the next day and human CFC generated after 3.5 weeks in culture were assayed as described. Support of human hematopoiesis in NOR-NOD SIRP wells was significantly better than in NOR-CEP wells (P=0.032 at 20 cells/well; P=0.008 at 2000 and 20,000 cells/well). Error bars represent standard deviations.

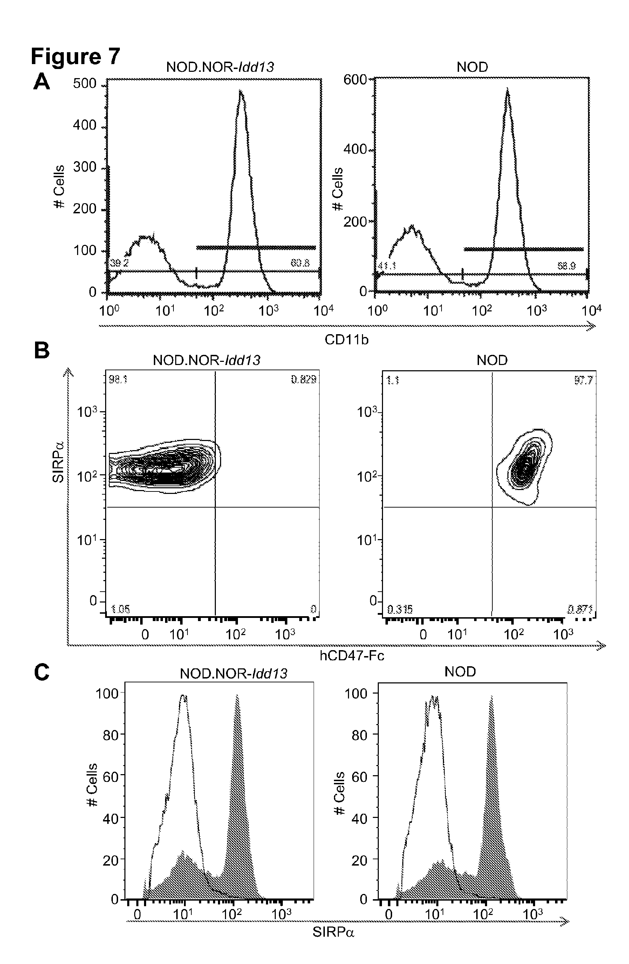

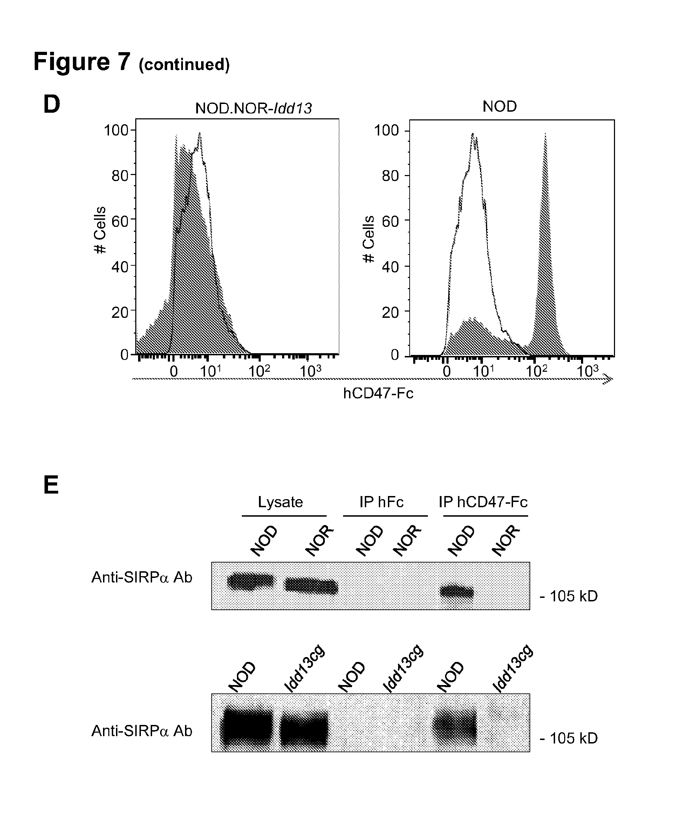

**Figure 7** illustrates conferral of enhanced cross species reactivity with human CD47 by NOD SIRPα. (a-d) Flow cytometric analysis of human CD47 (hCD47) binding to murine SIRPα on BM-derived macrophages from NOD and NOD.NOR-*Idd13* congenic mice. (a) Histogram of CD11b expression; horizontal bar indicates CD11b⁺ gate. Plots in b through d are gated on CD11b. (b) Two dimensional contour plots showing SIRPα and hCD47-Fc staining. (c) Histogram of SIRPα expression (grey) compared to isotype control (black). (d) Histogram of hCD47-Fc staining (grey) overlaid with human IgG-Fc control (black). (e) Immunoprecipitation of murine SIRPα with hCD47-Fc. Protein extracts from BM-derived macrophages from NOD, NOR and NOD.NOR-*Idd13* congenic mice were electrophoresed on SDS-PAGE and immunoblotted with polyclonal anti-SIRPα antibody. Each panel displays total lysate, immunoprecipitates with control human IgG-Fc protein (IP hFc) and immunoprecipitates with hCD47-Fc fusion protein (IP hCD47-Fc). The top panel compares NOD and NOR extracts, and the bottom panel compares NOD and NOD.NOR-*Idd13* (*Idd13cg*) extracts.

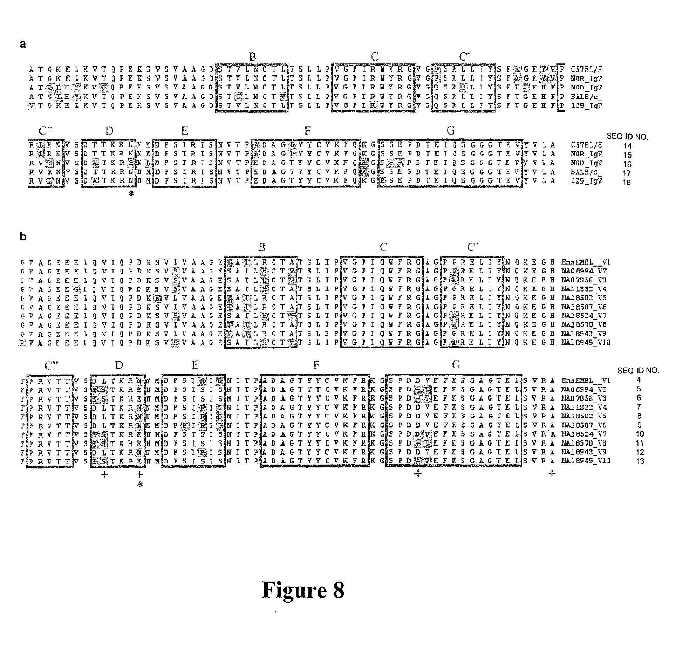

**Figure 8** illustrates protein sequence alignments of murine and human SIRPα IgV domains. (a) cDNA prepared from BM macrophages was used as a template for PCR amplification of Sirpα transcripts from NOD and NOR mice. The C57BL/6 (B6), BALB/c and 129/Sv sequences were obtained from EnsEMBL and NCBI databases. Open boxes represent b-pleated sheets identified in the X-ray crystal structure of SIRPα and correspond to similar regions in the Ig heavy chain variable region. Amino acids that vary between mouse strains are shaded. B6 was used as the parental sequence. (b) Exon 3 of SIRPα containing the IgV domain was PCR amplified from genomic DNA of 37 individuals from the human HapMal phase 1 release. Open boxed regions and shaded amino acids represent the same features as in (a), with V1 serving as the parental sequence. The residue marked * in an orthologous position between species, is polymorphic between NOD and other strains, and between humans. The residues marked + are polymorphic in humans and reside on the "top" solvent exposed surface of the protein where CD47 is predicted to bind.
**Figure 9** illustrates the validation of the discrimination power of three SNP assays by independent sequencing of the SIRPα IgV domain PCR amplified from each of the human HapMap samples shown (left side of the table).
**Figure 10** illustrates the CD47 protein including extracellular IgV-like loop, five membrane spanning regions and short cytoplasmic tail.
**Figure 11** illustrates (a) a histogram depicting protein production in the supernatant of cultured cells transfected with mCD47-Fc and two hCD47-Fc constructs prepared with different plasmid backbones (pcDNA and pIAP369) and (b) an immunoblot of an SDS-PAGE depicting the fusion proteins following purification from culture supernatant.
**Figure 12** illustrates (a) the introduction of a Kozak sequence into the pIAP369 plasmid construct containing mCD47-Fc and (b) the sequence hCD47-Fc inserted into the pIAP369 plasmid, showing the Kozak consensus, hCD47 fragment, linker and Fc.
**Figure 13** illustrates (a) the production of mouse and human CD47-Fc assayed in the supernatant of cells transfected with the Kozak containing plasmid and (b) an immunoblot displaying an anti-human Fc antibody reacting with the protein G-purified CD47-Fc proteins.
**Figure 14** illustrates an assay designed to quantify the binding affinity of hCD47-Fc and mCD47-Fc (not shown) to the NOD and NOR mouse versions of SIRPα.
**Figure 15** illustrates (a) results of binding of mCD47 to NOD and NOR SIRPα; and (b) results of binding of hCD47 to NOD and NOR SIRPα.
**Figure 16** is a schematic showing SIRPα-CD47 mediated signaling.
**Figure 17** shows the preparation of a truncated version of the human HSC "permissive" NOD version of SIRPα ("NOD-Δ-cyto") (left side).
**Figure 18** illustrates human HSC survival with NOR macrophages infected with an "empty" lentivirus (NOR-CEP "diamonds"), NOD macrophages not infected with virus (NOD uninf; filled triangles), NOD macrophages infected with "empty" lentivirus (NOD-CEP; circles), NOR macrophages infected with lentivirus containing full length NOD SIRPα (NOR-SIRP; squares), and NOR macrophages infected with lentivirus containing truncated NOD SIRPα (NOD-Δ-cyto; open triangles).
**Figure 19** illustrates a schematic of a study to evaluate *in vivo* the effect of CD47 fusion protein on HSC engraftment.

### DETAILED DESCRIPTION

In the following description, numerous specific details are set forth to provide a thorough understanding of the invention. However, it is understood that the invention may be practiced without these specific details.

As used herein "*conservative amino acid substitution*" refers to grouping of amino acids on the basis of certain common properties. A functional way to define common properties between individual amino acids is to analyze the normalized frequencies of amino acid changes between corresponding proteins of homologous organisms (Schulz, G. E. and R. H. Schirmer., Principles of Protein Structure, Springer-Verlag). According to such analyses, groups of amino acids may be defined where amino acids within a group exchange preferentially with each other, and therefore resemble each other most in their impact on the overall protein structure (Schulz, G. E. and R. H. Schirmer., Principles of Protein Structure, Springer-Verlag). Examples of amino acid groups defined in this manner include:
(i) a charged group, consisting of Glu and Asp, Lys, Arg and His,
(ii) a positively-charged group, consisting of Lys, Arg and His,
(iii) a negatively-charged group, consisting of Glu and Asp,
(iv) an aromatic group, consisting of Phe, Tyr and Trp,
(v) a nitrogen ring group, consisting of His and Trp,
(vi) a large aliphatic nonpolar group, consisting of Val, Leu and Ile,
(vii) a slightly-polar group, consisting of Met and Cys,
(viii) a small-residue group, consisting of Ser, Thr, Asp, Asn, Gly, Ala, Glu, Gln and Pro,
(ix) an aliphatic group consisting of Val, Leu, Ile, Met and Cys, and
(x) a small hydroxyl group consisting of Ser and Thr.

In addition to the groups presented above, each amino acid residue may form its own group, and the group formed by an individual amino acid may be referred to simply by the one and/or three letter abbreviation for that amino acid commonly used in the art.

As used herein, a "*cultured cell*" means a cell which has been maintained and/or propagated *in vitro.* Cultured cells include primary cultured cells and cell lines. As used herein, "*culturing the cell*" means providing culture conditions that are conducive to polypeptide expression. Such culturing conditions are well known in the art.

As used herein "*engrafting*" a stem cell, preferably an expanded hematopoietic stem cell, means placing the stem cell into an animal, e.g., by injection, wherein the stem cell persists *in vivo.* This can be readily measured by the ability of the hematopoietic stem cell, for example, to contribute to the ongoing blood cell formation.

As used herein "*fragment*" relating to a polypeptide or polynucleotide means a polypeptide or polynucleotide consisting of only a part of the intact polypeptide sequence and structure, or the nucleotide sequence and structure, of the reference gene. The polypeptide fragment can include a C-terminal deletion and/or N-terminal deletion of the native polypeptide, or can be derived from an internal portion of the molecule. Similarly, a polynucleotide fragment can include a 3' and/or a 5' deletion of the native polynucleotide, or can be derived from an internal portion of the molecule.

As used herein *"fusion protein"* refers to a composite polypeptide, i.e., a single contiguous amino acid sequence, made up of two (or more) distinct, heterologous polypeptides which are not normally fused together in a single amino acid sequence. Thus, a fusion protein may include a single amino acid sequence that contains two entirely distinct amino acid sequences or two similar or identical polypeptide sequences, provided that these sequences are not normally found together in the same configuration in a single amino acid sequence found in nature. Fusion proteins may generally be prepared using either recombinant nucleic acid methods, i.e., as a result of transcription and translation of a recombinant gene fusion product, which fusion comprises a segment encoding a polypeptide of the invention and a segment encoding a heterologous polypeptide, or by chemical synthesis methods well known in the art. Fusion proteins may also contain a linker polypeptide in between the constituent polypeptides of the fusion protein. The term "*fusion construct*" or "*fusion protein construct*" is generally meant to refer to a polynucleotide encoding a fusion protein. In one embodiment, the fusion protein is a polypeptide as described herein fused to a portion of an Ig molecule. The Ig portion of the fusion protein can include an immunoglobulin constant region, e.g. a human Cγ1 domain or a Cγ4 domain (e.g. the hinge, CH2, and CH3 regions of human IgγC1 or human IgCγ4 (see e.g., Capon et al., U.S. Pat. Nos. 5,116,964; 5,580,756; 5,844,095, and the like). In one preferred embodiment, Ig fusion proteins include a polypeptide as described herein coupled to an immunoglobulin constant region (e.g., the Fc region).

As used herein "*hematopoietic stem cell*" refers to a cell of bone marrow, liver, spleen or cord blood in origin, capable of developing into any mature myeloid and/or lymphoid cell.

As used herein "*isolated*" with reference to a nucleic acid molecule, polypeptide, or other biomolecule, means that the nucleic acid or polypeptide has separated from the genetic environment from which the polypeptide or nucleic acid were obtained. It can also mean altered from the natural state. For example, a polynucleotide or a polypeptide naturally present in a living animal is not "*isolated*," but the same polynucleotide or polypeptide separated from the coexisting materials of its natural state is "*isolated*", as the term is employed herein. Thus, a polypeptide or polynucleotide produced and/or contained within a recombinant host cell is considered isolated. Also intended as an "*isolated polypeptide*" or an "*isolated nucleic acid molecules*" are polypeptides or nucleic acid molecules that have been purified, partially or substantially, from a recombinant host cell or from a native source.

It is also contemplated that the peptides of the invention may exhibit the ability to modulate biological, such as intracellular, events. As used herein "*modulate*" refers to a stimulatory or inhibitory effect on the biological process of interest relative to the level or activity of such a process in the absence of a peptide of the invention.

The term "*macrophage suppression*" or "*suppression of macrophages*" as used herein refers to the reduction or prevention of the role, activity and/or effect of macrophages. As used herein, the "*nucleic acid molecule*" means DNA molecules (e.g., a cDNA) and RNA molecules (e.g., an mRNA) and analogs of the DNA or RNA generated, e.g., by the use of nucleotide analogs. The nucleic acid molecule can be an oligonucleotide or polynucleotide and can be single-stranded or double-stranded.

As used herein "*operably linked*" refers to an arrangement of elements wherein the components so described are configured so as to perform their usual function. Thus, control elements operably linked to a coding sequence are capable of effecting the expression of the coding sequence. The control elements need not be contiguous with the coding sequence, so long as they function to direct the expression thereof. Thus, for example, intervening untranslated yet transcribed sequences can be present between a promoter and the coding sequence and the promoter can still be considered "*operable linked*" to the coding sequence. Similarly, "control elements compatible with expression in a subject" are those which are capable of effecting the expression of the coding sequence in that subject.

As used herein, "*pharmaceutically acceptable carrier*" means any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the pharmacological agent.

As used herein, "*polypeptide*" and "*protein*" are used interchangeably and mean proteins, protein fragments, modified proteins, amino acid sequences and synthetic amino acid sequences. The polypeptide can be glycosylated or not.

As used herein "*stroma*" refers to the supporting tissue or matrix of an organ, distinguishable from the functional elements of the organ or the parenchyma.

As used herein, "*therapeutically effective amount*" refers to an amount effective, at dosages and for a particular period of time necessary, to achieve the desired therapeutic result. A therapeutically effective amount of the pharmacological agent may vary according to factors such as the disease state, age, sex, and weight of the individual, and the ability of the pharmacological agent to elicit a desired response in the individual. A therapeutically effective amount is also one in which any toxic or detrimental effects of the pharmacological agent are outweighed by the therapeutically beneficial effects.

According to one aspect, there is provided an isolated polypeptide selected from the group consisting of a polypeptide consisting of a) the amino acid sequence of SEQ ID NO. 1; b) a polypeptide consisting of a fragment of the amino acid sequence of SEQ ID NO. 1, wherein the fragment comprises at least one of residues 31, 32, 34, 37, 74, 77, 83, 84, 86, 87, 90, 91, 96, 100, 102, 114, 118, 126 of SEQ ID NO. 1; and c) one of the polypeptide in a) and b) with up to 1 amino acid insertion, deletion or substitution for every 7 amino acids in length of the polypeptide, wherein the polypeptide comprises at least one of residues 31, 32, 34, 37, 74, 77, 83, 84, 86, 87, 90, 91, 96, 100, 102, 114, 118, 126 of SEQ ID NO. 1 and binds human CD47.

According to a further aspect, there is provided an isolated polypeptide selected from the group consisting of a) a polypeptide consisting of the amino acid sequence of SEQ ID NO. 2; b) a polypeptide consisting of a fragment of the amino acid sequence of SEQ ID NO. 2; and c) one of the polypeptide in a) and b) with up to 1 amino acid insertion, deletion or substitution for every 7 amino acids in length of the polypeptide; wherein i) at least one of residues at positions 31, 32, 34, 37, 74, 77, 83, 84, 86, 87, 90, 91, 96, 100, 102, 114, 118, 126 of SEQ ID NO. 2 in the polypeptide is replaced with corresponding residues 31, 32, 34, 37, 74, 77, 83, 84, 86, 87, 90, 91, 96, 100, 102, 114, 118, 126 of SEQ ID NO. 1; or ii) at least one of residues 129 and 130 of SEQ ID NO. 2 in the polypeptide is deleted.

According to a further aspect, there is provided an isolated polypeptide selected from the group consisting of a) a polypeptide consisting of an amino acid sequence selected from the group consisting of SEQ ID NOS. 4-13; b) a polypeptide consisting of a fragment of an amino acid sequence selected from the group consisting of SEQ ID NOS. 4-13; and c) one of the polypeptide in a) and b) with up to 1 amino acid insertion, deletion or substitution for every 7 amino acids in length of the polypeptide, wherein the polypeptide binds human CD47.

According to a further aspect there is provided a polypeptide capable of binding to human Sirpα; and antibodies to human Sirpα. Preferably, the polypeptide is the extracellular domain of human CD47 fused to the Fc portion of IgG.

In some embodiments, the polypeptide is the fragment thereof and comprises at least 3 consecutive amino acids in at least one of a region between residues 50-57, 63-71, 74-80, 88-92, 95-100, 103-109, 114-125 or 128-141, inclusive of SEQ ID NO. 1, between residues 50-57, 63-71, 74-80, 88-92, 95-100, 103-109, 114-125 or 128-143, inclusive of SEQ ID NO. 2, between residues 24-31, 37-45, 48-54, 62-66, 69-74, 77-83, 88-99 or 102-116, inclusive, of any one of SEQ ID NOs. 4, 7, 8, 9 and 12; or between residues 24-31, 37-45, 48-54, 62-66, 69-74, 77-83, 88-99 or 102-115, inclusive, of SEQ ID NOs. 5, 6, 10, 11 and 13, as the case may be.

In some embodiments, the amino acid insertion, deletion or substitution is a conservative amino acid substitution.

In a preferred embodiment, the polypeptide binds human CD47.

In a preferred embodiment, the polypeptide binds human SIRPα.

In some embodiments, in increasing preferability, the polypeptide is the fragment and is between 6 and 30 amino acids in length, between 8 and 28 amino acids in length, between 10 and 26 amino acids in length, between 12 and 24 amino acids in length and between 14 and 22 amino acids in length.

The polypeptides described herein may be fused to a second polypeptide that is preferably the Fc portion of IgG.

According to one preferred embodiment, there is provided a polypeptide comprising the extracellular domain of CD47 fused to the Fc portion of IgG, preferably for increasing hematopoietic stem cell engraftment in a human.

According to a further aspect, there is provided a pharmaceutical composition comprising a polypeptide described herein and a pharmaceutically acceptable carrier.

According to a further aspect, there is provided a method for increasing hematopoietic stem cell engraftment in a mammal comprising administering to the mammal a therapeutically effective amount of a polypeptide described herein. Preferably, the increased hematopoietic stem cell engraftment results from suppression of macrophages.

According to a further aspect, there is provided a use of a polypeptide described herein for increasing hematopoietic stem cell engraftment in a mammal or in the preparation of a medicament for increasing hematopoietic stem cell engraftment in a mammal.

According to a further aspect, there is provided a polypeptide described herein for increasing hematopoietic stem cell engraftment in a mammal.

According to a further aspect, there is provided an isolated nucleic acid comprising a sequence that encodes a polypeptide described herein.

According to a further aspect, there is provided an expression vector comprising a nucleic acid described herein, preferably comprising a Kozak consensus.

According to a further aspect, there is provided a cultured cell comprising a vector described herein.

According to a further aspect, there is provided a method of producing a polypeptide comprising culturing a cell described herein under conditions permitting expression of the polypeptide.

According to a further aspect, there is provided a method for increasing hematopoietic stem cell engraftment in a human comprising modulating the interaction between human Sirpα and human CD47. Preferably, the interaction between human Sirpα and human CD47 is modulated by administering a therapeutically effective amount of at least one of a) a polypeptide capable of binding to the extracellular domain of human Sirpα; b) antibodies to human Sirpα ; c) a polypeptide capable of binding to the extracellular domain of human CD47; and d) antibodies to human CD47. Correspondingly, there is also provided use of the foregoing for increasing hematopoietic stem cell engraftment in a human or for preparing medicament for the same. Preferably, the increased hematopoietic stem cell engraftment results from suppression of macrophages.

In one embodiment, the polypeptide capable of binding to the extracellular domain of human Sirpα comprises soluble human CD47, or a fragment thereof, preferably the extracellular domain of CD47. Preferably, the soluble human CD47, or a fragment thereof, is fused to a second protein. In another embodiment, the polypeptide capable of binding to the extracellular domain of human CD47 comprises soluble human Sirpα, or a fragment thereof, preferably the extracellular domain of Sirpα. Preferably, the soluble human Sirpα, or a fragment thereof, is fused to a second protein. In preferred embodiments, the second protein is the Fc portion of IgG.

According to a further aspect, there is provided a method of identifying a compound that increases hematopoietic stem cell engraftment in a human comprising a) contacting at least one of the extracellular domain of human Sirpα and human CD47 with at least one test compound; b) determining the at least one test compound as binding to the at least one of human Sirpα and human CD47; c) contacting the test compound with human hematopoietic cells in a stromal environment; and d) determining whether hematopoietic stem cell engraftment is increased in the presence of the test compound.

According to a further aspect, there is provided a method of determining genetic polymorphisms in humans affecting hematopoietic stem cell engraftment comprising a) sequencing the Sirpα gene from a plurality of humans having undergone hematopoietic transplantation; b) determining nucleotide differences in the Sirpα gene within the plurality of humans; and c) correlating the nucleotide differences with hematopoietic stem cell engraftment to determine relevant polymorphisms. Preferably, the nucleotide differences result in amino acid differences.

According to a further aspect, there is provided a method of determining likelihood of hematopoietic stem cell engraftment in a recipient comprising a) sequencing the Sirpα gene from the recipient; and b) determining whether the relevant polymorphisms exist. Preferably, the relevant polymorphisms result in an amino acid difference that is preferably at least one of (a) replacement of at least one of residues at positions 31, 32, 34, 37, 74, 77, 83, 84, 86, 87, 90, 91, 96, 100, 102, 114, 118, 126 of SEQ ID NO. 2 with corresponding residues 31, 32, 34, 37, 74, 77, 83, 84, 86, 87, 90, 91, 96, 100, 102, 114, 118, 126 of SEQ ID NO. 1; or (b) deletion of at least one of residues 129 and 130 of SEQ ID NO. 2.

The advantages of the present invention are further illustrated by the following examples. The examples and their particular details set forth herein are presented for illustration only and should not be construed as a limitation on the claims of the present invention.

### EXAMPLES

### MATERIALS & METHODS

### Mice

The CB17*-scid*/*scid* (SCID), NOD/LtSz*-Prdkc*^{*sc*/*sc*} (NOD.*SCID*) (Shultz, L.D. et al. (1995) J Immunol 154, 180-91), C57BL/6 (B6), C3H, ICR, and BALB/c mice were bred and maintained under sterile conditions at the Ontario Cancer Institute (Toronto, Canada). *Rag-2*^{*-*/-} (RAG-2), RAG-2.*Prf*^{*-*/-}, and RAG-2.β*₂m*^{-/-} mice, all on B6 background, were obtained from GenPharm International (Mountain View, CA). NOD, *NOD.SCID,* NOD.NOR-*Idd13*, NOD.NOR-*Idd4*, NOD.NOR-*Idd5*, NOD.NOR-*Idd9*, NOR.NOD-*Idd13*, and B6.NOD-*Idd13* mice were maintained in either specific pathogen-free or barrier conditions at the Hospital for Sick Children (Toronto, Ontario).

All congenic mice were generated with marker-directed selection of breeders from an initial intercross of NOD/Jsd and NOR/Lt progenitors. The NOD and NOR genomes are approximately 88% identical by descent, and the differential portions of their genomes are distributed on chromosomes 1, 2, 4, 5, 7, 10, 11, 12, 14 and 18. Breeders were screened with microsatellite markers for all genetic loci that differ between NOD and NOR, such that the founders of all congenic lines were homozygous for background alleles. The microsatellite markers used to define the congenic mice used in this study are listed in (Table 1.

**Table 1. Microsatellite markers used to map congenic mice.**

| **Markers used to define congenic intervals** | | | | | | |
|---|---|---|---|---|---|---|
| Locus | Marker | Chr. | Position (Mb) | Primer 1 | Primer 2 | SEQ ID NO. |
| *IBD* | *D1Gul11 7* | 1 | 72614682 | GAACATAGGTGCACACTC ACATA | TGATGCTGGAGGTTGTCC T | 19/20 |
| *Idd5* | *D1Ngul4 6* | 1 | 72932895 | GATGAAAGAGATAAGAC AGATGTTAGATA | TGGTTATTAAAGAGCTGT TTTACATG | 21/22 |
| *Idd5* | *D1Mit77* | 1 | 74154726 | AAGTTGGAACTCTGCAGG ACA | GTGTCTTCAATGCAGCAC GT | 23/24 |
| *Idd5* | *D1Mit13 2* | 1 | 77603656 | TATTGTTTATGGAAATTG GACCC | CATCTCTGAAGGAAAAA GTGCA | 25/26 |
| *Idd5* | *D1Mit21 6* | 1 | 80318888 | GGGAGACAACAAATAAT CATATTGC | AGAGGTGGGTCCTGGAA ACT | 27/28 |
| *Idd5* | *D1Mit8* | 1 | 83489415 | CTGAAAATCGTCCCTTGA CC | CAGGAGCATGAAATGGG GAT | 29/30 |
| *Idd5* | *D1Mit44 0* | 1 | 88698913 | TCCACACAAGGTGTCCTC TG | GCTCAGGTGACCTCCAA AAC | 31/32 |
| *Idd5* | *Ramp1* | 1 | 91094432 | ATCACCACTGTGGGCATT CTGAAC | CAGTAGAGGCCAAGGGC ATCAGAATC | 33/34 |
| *IBD* | *D1Mit84* | 1 | 91834941 | TGTCTCCCCAAAGTAGCA GG | GTGATGCAGGAGTTTCT GCA | 35/36 |
| *IBD* | *D2Jyh43 9* | 2 | 11898874 8 | GGCAGATTTATCGCTGTG AG | GTGCGCCACCAATGC | 37/38 |
| *Idd13* | *D2Jyh44 3* | 2 | 11904730 1 | CTTCAGTGCTGTATCCAC TGC | CTCCCACTCCCATTAATT CTC | 39/40 |
| *Idd13* | *D2Mit17* | 2 | 12256306 9 | AGGCAATTACAAGGCCTG G | CACCCATCTCCCTCAGTC AT | 41/42 |
| *Idd13* | *D2Mit25 6* | 2 | 12636563 4 | GTAGAGACTTGGTGGGTT TTGG | ATCTCTGCTGACAAAGA ACAGTG | 43/44 |
| *Idd13* | *D2Mit44 7* | 2 | 12853044 2 | CATGTGCCATGGTACAAA GC | TGGCTTGTTTCAGATAGC ACA | 45/46 |
| *Idd13* | *D2Mit33 8* | 2 | 13057830 9 | TCACCAGCCTGAAAACAC TG | TCTGGGTACAATCCTTAG TCCTG | 47/48 |
| *Idd13* | *D2Mit49 0* | 2 | 13864439 8 | GCCAGATACAATTTTCCC TCA | GTGGGACGCAATATTGG C | 49/50 |
| *Idd13* | *D2Mit45 2* | 2 | 16245001 5 | TCCCACATTTCTGGCATA CA | CACATGTGCATTTAAGC ATGC | 51/52 |
| *IBD* | *D2Mit51* | 2 | 16321366 1 | GTGAGGGGTCAATGCCAC | GGCTCAGTTGTAAGCAC AAGG | 53/54 |
| *IBD* | *D4Mit33 1* | 4 | 10224868 2 | CCTAACCCTCCCCACACC | AAAGATCTGGATTCAAA TCCTCC | 55/56 |
| *Idd9* | *D4Mit31* | 4 | 10545792 0 | ACGAGTTGTCCTCTGATC AACA | AGCCAGAGCAAACACCA ACT | 57/58 |
| *Idd9* | *D4Mit30 8* | 4 | 12253146 4 | TATGGATCCACTCTCCAG AAA | CAAAGTCTCCTCCAAGG CTG | 59/60 |
| *Idd9* | *D4Mit20 4* | 4 | 13188076 3 | CTGCTGCAGCGATTCTCT C | TCAGGCACCTAAGTACA TGTGC | 61/62 |
| *Idd9* | *D4Mit25 1* | 4 | 13538496 3 | AAAAATCGTTCTTTGACT TCTACATG | TTTAAAAGGGTTTCTTTA TCCTGTG | 63/64 |
| *Idd9* | *D4Mit25 9* | 4 | 14277093 7 | TTTTCTTAGAACATACCT TGCTTGG | GGGGAAGGTGAAAATTT AAAGG | 65/66 |
| *Idd9* | *D4Mit31 0* | 4 | 14658249 7 | TCTCCACGTGTGTGCCTT AG | TGAAAGCACTCTGCAGA CTCA | 67/68 |
| *Idd9* | *D4Mit18 9* | 4 | 14699441 1 | CACTACACGGGGCTGAG ATT | AAGATCCAACACCCTTTT TGG | 69/70 |
| *IBD* | *D4Ngul7 383* | 4 | 14777240 5 | GGCAGCTTTGTGCTTTAA AA | ATGGATGTTTACCTGGCG T | 71/72 |
| *Idd4* | *D11Mit2 17* | 11 | 37214368 | ACTGGAAAATATGTTTTA AACCTCTG | AAATGGGATTCTGCAAA AACC | 73/74 |
| *Idd4* | *D11Mit3* | 11 | 53814488 | GTGCACTTTCCATGCCTG | GAGTAGAAAGAGACAGA | 75/76 |
| | *10* | | | TA | GAAAGACACA | |
| *Idd4* | *D11Mit9 0* | 11 | 70112196 | TCTCCAGCCCCTTCATTA TG | TGCCAAACACCCATGAG AC | 77/78 |
| *Idd4* | *D11Jyh1 081* | 11 | 80495333 | TCCTTGGTGAGTCCACAT CT | GGCTAGGCTCTGGAAGA CA | 79/80 |
| *IBD* | *D11Mit4 2* | 11 | 11279582 4 | ACTAGCCATATGGTTTCT GATGG | GTAGCAGGGCTGTGAGC TTT | 81/82 |
| | | | | | | |
| | | | | | | |

| **Markers used to screen for background alleles in congenic mice** | | | | | | |
|---|---|---|---|---|---|---|
| locus | marker | Chr. | position (cM) | primer1 | primer2 | SEQ ID NO. |
| *non-Idd* | *D1Mit430* | 1 | 6.6 | TATTAATGTTGAAGCCA GAAGCC | CTTTAATCATCTCTGTGG CAAGG | 83/84 |
| *non-Idd* | *D1Mit122* | 1 | 20.4 | TGAAGCAAGAATTCAA GAAGAGG | AGGACATACCTAGTAAG GCTTTGTC | 85/86 |
| *non-Idd* | *D5Mit11* | 5 | 17.5 | GATCTTCCTACCTTCTTA CCCAC | CATGATTTTATTTGGGGG G | 87/88 |
| *non-Idd* | *D7Mit124* | 7 | 37.2 | GTCAGACATTGGCTTAG GATCC | GGTTTGTGCGCTCTCTCT CT | 89/90 |
| *non-Idd* | *D10Mit86* | 10 | 12 | TTTGCCTGTAACAAGCC AGA | TTGAGGCTATCAGTTTAA AATCCC | 91/92 |
| *non-Idd* | *D11Mit62* | 11 | 2.2 | GAATAACCCATGTTTAT ATCGGTG | CTCTGGACTTGTGTTCTA TGCC | 93/94 |
| *non-Idd* | *D12Mit76* | 12 | 38.3 | CCTCCTAATAGTGCCAT TCCC | CCAGCAATCACGAGTCT TCC | 95/96 |
| *non-Idd* | *D14Mit194* | 14 | 52.5 | GACCAAAGTCATATCTA TAACACC | TTAATTGCAAGTAACAC AATGAGTAGG | 97/98 |
| *non-Idd* | *D18Mit4* | 18 | 37.2 | ACTGTTGCTGGGGAATG G | CCAAGTTCAAAGCTGCT GG | 99/100 |

| | | | | | | |
|---|---|---|---|---|---|---|
| IBD = identical by descent; non-*Idd* = regions of non-identity between NOD and NOR outside the four *Idd* regions | | | | | | |

NOD.NOR-*Idd13.SCID* mice were generated from an intercross of NOD.NOR-*Idd13* mice to NOD.*SCID* mice, followed by brother-sister matings screened by marker assisted genotyping until homozygosity was achieved at both *Idd13* and *SCID* loci.

For microsatellite genotyping, genomic DNA was prepared from 0.6 - 0.8 cm tail snips that were digested in a solution of proteinase K and digestion buffer (AutoGen AG00122) overnight at 55°C. The crude digests were diluted and heated to inactivate the proteinase K. PCR was performed with oligonucleotide primers modified at their 5' ends with fluorescent tags. PCR conditions were the same for all microsatellite primers: 10 cycles of 30 s at 94°C, 30 s at 50°C, and 1 min at 72°C, followed by 35 cycles of 30 s at 94°C, 30 s at 55°C, and 1 min at 72°C in 1.5 mM MgCl₂. PCR amplicons were detected with the ABI 3730XL sequencer and alleles were called with the aid of the GeneMapper software version 3.0 (Applied Biosystems, Foster City, CA).

For LTC on MS-5 murine stromal cells, non-irradiated MS-5 cells were seeded into 96 well tissue culture plates (2000 cells/well) as described in Gan, O.I. et al. (1999) Exp Hematol 27, 1097-106. Three days later, murine peritoneal macrophages were seeded at doses of 20-20,000 cells/well with 5 replicates per dose. The next day, 2000 human Lin CB cells per well were added in 200 µl human LTBMC medium without hydrocortisone. Cultures were maintained at 33°C (or 37°C for gene transfer experiments) with weekly half-medium change. After 3-4 weeks, cells were trypsinized and half of the cells from each well were plated into progenitor assays as described.

### Human-specific hematopoietic progenitor cell assay

Seven thousand five hundred cells from T25 flasks, or half well content from 96-well plates, were plated in 1 ml methylcellulose cultures under conditions that are selective for human cells as previously described in Larochelle, A. et al. (1996) Nat Med 2, 1329-37. The cultures were incubated for 14 days at 37°C and the total number of colonies produced by colony-forming cells (CFC) was counted. Cells from T25 flasks were plated in triplicate.

### Murine macrophage preparation

Murine peritoneal macrophages were obtained by peritoneal levage with 10 ml of αMEM with 10% FCS. BM-derived macrophages were obtained by flushing femurs, tibias, and iliac crests with 10 ml of DMEM supplemented with 10% FCS, 10 mM HEPES (pH 7.0), 50 nM 2-mercaptoethanol, 2 mM glutamine, 1× nonessential amino acids, and 1% penicillin/streptomycin ("complete DMEM"). BM cells were plated at a concentration of 1 × 10⁶ cells/ml in complete DMEM containing 10 ng/ml recombinant mouse GM-CSF (R&D Systems, Minneapolis, MN). Medium was changed with fresh complete DMEM with GM-CSF every other day, and macrophages were harvested by gentle scraping after 7 days.

### Real time PCR analysis of Idd13 candidate genes

Expression of genes within the 0.96 Mb critical *Idd13* interval was analyzed by PCR amplification of cDNA prepared from BM stroma and purified macrophages. Real time PCR was performed using an ABI/PRIZM 7900 HT cycler (Applied Biosystems) under the following conditions: 95°C for 15 s and 59°C for 1 min for 40 cycles, with addition of SYBR Green buffer (Applied Biosystems). Serial dilutions of a plasmid containing the gene sequence were used as a reference for standard curve calculation. The fluorescence thresholds and corresponding copy number were calculated using SDS 2.0 software (Applied Biosystems). Reactions were performed in triplicate, and transcript quantity was normalized to β-actin.

### Molecular genetic analysis of murine Sirpα

The coding region of *Sirp*α was sequenced from NOD, NOR and BALB/c strains. cDNA was prepared from BM-derived macrophages and used as a PCR template under the following conditions: 30 s at 94°C, 30 s at 60°C, and 1 min at 68°C in 2 mM MgSO₄ for 30 cycles using High-Fidelity Taq polymerase (Invitrogen Life Technologies, Carlsbad, CA). The untranslated (UTR) region of *Sirp*α was also amplified and sequenced in NOD and NOR strains, using genomic DNA as a template and the same PCR conditions. Sequencing primers were designed using Primer Express 1.5 software (Applied Biosystems) and are listed in Table 2. PCR products were purified according the manufacturer's instructions (Qiagen, Valencia, CA) and sequenced on both strands at the Center for Applied Genomics, Hospital for Sick Children, Toronto, Canada (TCAG; www.tcag.ca). B6, BALB/c, and 129/Sv mouse *Sirp*α sequence files were obtained from EnsEMBL (www.ensembl.org) and NCBI (www.ncbi.nlm.nih.gov) databases. Sequence files were aligned and analyzed with Lasergene software (DNASTAR, Madison, WI).

**Table 2. Primers used to PCR amplify and sequence coding and untranslated regions of murine and human SIRPα.**

| | Forward Primer | Reverse Primer | SEQ ID NO. |
|---|---|---|---|
| Mouse | | | |
| Sirpα-1 | AGTCCACCTTAAGAGGACCA AGTAGC | TGTACAGAAACAGGACGCGGA | 101/102 |
| Sirpα-2 | TCTCCCTCCTTGCTCTGCAG | TACCTCGCAGATGACCTTAGA ATTAA | 103/104 |
| Sirpα-3 | CCTAGTGGAAAGAATGTCTCC TACAACA | CATATTCTGTGTGGTTGTTAGG CTCA | 105/106 |
| Sirpα-4 | CCAGGTACAGTCTTTGATCCA GGA | CCAGGGAGTCTCTGCTGGTCT A | 107/108 |
| Sirpα-5 | CATCTGTGGAAGAACTACAC CAGAAGTC | TACCTCAGAGAGCTGGCCGAG T | 109/110 |
| Sirpα-6 | CCAATGCTGACCTAATGTTGG C | CATCTATACACCCGTGTGTGTA TACACA | 111/112 |
| Sirpα-7 | AACTTGTCTTTGTCCGGGCC | GGCACAGTTCATCCTCACCC | 113/114 |
| | | | |
| Human | | | |
| Sirpα | TAGAATACAGGCTCATGTTGC AGGT | GCCTTCAGCAAATAGCATGAC GT | 115/116 |

### Molecular genetic analysis of human SIRPα

Thirty-seven unrelated individuals from the HapMap collection were chosen for sequencing based on genotype variation at two markers flanking the IgV domain encoded in exon 3 (rs6075340 and rs611968). DNA samples were obtained from TCAG and exon 3 was amplified by PCR using High-Fidelity Taq polymerase (Invitrogen Life Technologies). The resulting amplicons were cloned and exon 3 regions from 2 to 4 independent clones per individual were sequenced at TCAG on both strands using primers flanking the plasmid multiple cloning site (Table 2). Sequences were aligned using Lasergene software.

### Biochemical analysis of SIRPα

BM-derived macrophages were washed with ice-cold PBS and then lysed in buffer (20 mM Tris-HCl pH 7.6, 140 mM NaCl, 1 mM EDTA, 1% NP-40) containing 5 mM NaF, aprotinin (10 µg/ml) and 1 mM sodium vanadate. The lysates were centrifuged at 10 000 g for 15 min at 4°C and the resulting supernatants subjected to immunoblot analysis as follows: proteins (20 µg per well) were electrophoresed in 10% sodium dodecyl sulphate (SDS)-polyacrylamide gels and transferred onto 0.2 µM nitrocellulose membranes (Bio-Rad, Hercules, CA). Western blots were analyzed using anti-SIRPα antibody (Stressgen, Victoria, BC, Canada) and developed with the HRP-ECL system (Amersham Biosciences, Piscataway, NJ). N-linked oligosaccharides were removed from SIRPα by boiling cell lysates for 5 minutes in 0.5% SDS and 1% βmercaptoethanol to denature the glycoproteins, and then incubating with N-glycosidase F (40 units/ml, Roche Applied Science, Indianapolis, IN) for 12 hours at 37°C. For immunoprecipitation, 250 µg of BM-derived macrophage lysate was added to either hCD47-Fc (10µg/ml) or the control human IgG Fc (10µg/ml) with 50µl of protein G MicroBeads (Miltenyi Biotec, Auburn, CA). Following a 60-minute incubation on ice, the immune complexes were collected on a µMACS separator following manufacturer's instructions (Miltenyi Biotec, Auburn, CA) and eluted from the column with SDS-PAGE gel loading buffer.

### Sirpα lentiviral infection protocol

NOD *Sirp*α cDNA was cloned downstream of an E1Fα promoter in a third-generation lentiviral vector backbone containing a reporter *Gfp* gene driven by the human PGK promoter (SIRP). The control vector (CEP) contained only *Gfp.* Vector construction was confirmed by sequencing. Viruses pseudotyped with the vesicular stomatitis virus G protein were generated by transient transfection of 293T cells, as described in Guenechea, G. et al. (2000) Mol Ther 1, 566-73, and concentrated by ultracentrifugation. The functional titers of SIRP and CEP virus as determined by infection of HeLa cells were 2.1-5.1 × 10⁸ and 4-7.6 × 10⁸ infectious particles per ml, respectively. Virus was added to flasks of NOR BM-derived macrophages after 5 days of culture at a multiplicity of infection of 20 to 25, and infected macrophages were harvested on day 12. Gene transfer efficiency into macrophages was 51-88% as assessed by GFP fluorescence using flow cytometry. Infected macrophages were stained with PE-conjugated anti-mouse CD11b mAb (Beckman Coulter, Fullerton, CA) and sorted on a Dako Cytomation MoFlo (Fort Collins, CO) to obtain GFP⁺CD11b⁺ cells. Uninfected control macrophages were stained and sorted to obtain CAD11b⁺cells. Purified macrophages (purity 97-99%) were used for further experiments.

### Soluble human CD47-Fc preparation

293T cells were transfected with pcDNA-CD47Fc containing the human CD47 extracellular domain fused to human IgG1 Fc directed by the CMV promoter (Latour, S. et al. (2001) J Immunol 167, 2547-54) using a Superfect Transfection Kit (Qiagen) and selected with 100µg/ml Zeocin (Invitrogen, Carlsbad, CA). Cell lines producing soluble human CD47-Fc (hCD47-Fc) were identified by immunoblot analysis of culture supernatants. hCD47-Fc protein was purified on Protein G-Sepharose (Pierce, Rockford, IL) and covalently conjugated to biotin for use in flow cytometric analyses.

### Flow cytometry

Flow cytometric analysis was performed using a FACSCalibur (BD, Franklin Lakes, NJ). For analysis of human cell engraftment in mice, cells harvested from murine BM were stained with phycoerythrin (PE) cyanine5-conjugated anti-human CD45 antibody (Beckman Coulter). For analysis of cell surface expression of SIRPα and binding of hCD47-Fc, 1 × 10⁶ murine BM leukocytes were stained with purified anti-SIRPα mAb P84 (BD) with Alexa 633-conjugated goat anti-rat (Molecular Probes, Carlsbad, CA), FITC-conjugated anti-CD11b mAb (SWRI, San Antonio, TX), and either biotinylated hCD47-Fc with avidin-APC or a control human IgG Fc fragment with biotinylated anti-human IgG mAb and avidin-APC. Viable leukocytes were selected by exclusion of propidium iodide. Isotype controls were mouse or rat IgG conjugated to the appropriate fluorochrome. GFP fluorescence was detected in FL1 calibrated to the fluorescein isothiocyanate emission profile.

### Statistical analysis

Groups were compared using one-way ANOVA and all pairwise multiple comparisons were done using Dunn's method when differences were found. For gene transfer **experiments, CEP and SIRP conditions were compared using the Wilcoxon rank test.**

### Protein Synthesis

The human *CD47 IgV* domain cDNA sequence was cloned in-frame upstream of a human *IgG Fc* cDNA sequence, inserted into appropriate expression vectors, transfected into 293F cells, and the resulting fusion protein (hCD47-Fc) purified from culture supernatant by affinity chromatography on protein G beads. Mouse CD47-Fc (mCD47-Fc) fusion protein was prepared using the same strategy.

### Assay of binding affinity of hCD47 and mCD47 to mSIRPα expressed on mouse macrophages

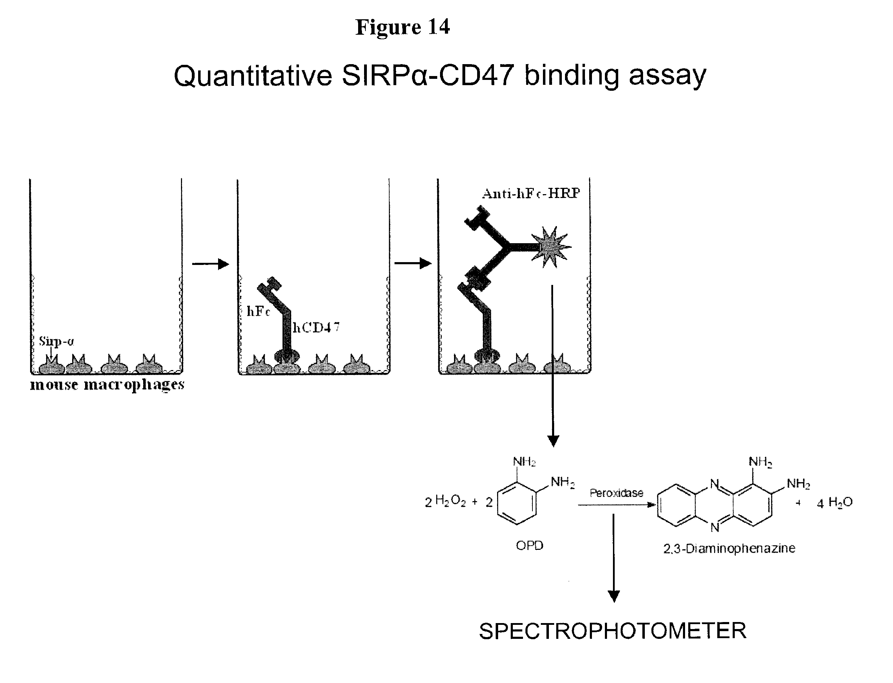

Figure 14 is a schematic representation of an assay designed to quantify the binding affinity of hCD47-Fc and mCD47-Fc (not shown) to the NOD and NOR mouse versions of SIRPα. Macrophages obtained from NOD or NOR mice were allowed to adhere to tissue culture plates for 2 hours then washed with buffered saline. Graded amounts of purified hCD47-Fc or mCD47-Fc fusion protein was added to the wells, allowed to bind at 37°C for 20 minutes, and the wells washed extensively with buffered saline. A commercially available anti-human Fc antibody covalently conjugated to horseradish peroxidase (HRP) was added and allowed to bind, and the plates washed extensively with buffered saline. The quantity of bound hCD47 was evaluated by colorimetric assay following HRP reaction with hydrogen peroxide and peroxidase treatment using a spectrophotometer.

### EXAMPLE 1

### Immune-deficient mice with NOD strain background support human hematopoietic cell engraftment in vivo

Several groups including our own have shown that NOD mice homozygous for the *Prkdc^{scid} (SCID)* mutation permit superior human hematopoietic cell engraftment compared to *SCID* mice on other strain backgrounds, including CB17 (Greiner, D.L. et al. (1995) Am J Pathol 146, 888-902; Larochelle, A. et al. (1996) Nat Med 2, 1329-37). The molecular and cellular basis for this strain difference in xenograft efficacy is not known. However, NOD.*SCID* mice have a high incidence of spontaneous thymic lymphoma that limits their usefulness for long-term experiments. Mice carrying null alleles of the recombinase activating gene 2 (*Rag-2*) on the C57BL/6 (B6) background have similar blockade in T and B cell development to CB17.*SCID* mice but do not display spontaneous lymphoid malignancies (Shinkai, Y. et al. (1992) Cell 68, 855-67). *Rag-2*^{*-*/*-*} (RAG-2) mice also carrying homozygous null alleles in β₂ microglobulin (RAG-2.β*₂m*^{*-*/-}) or perforin (RAG-2.*Prf*^{*-*/*-*}) possess additional defects in natural killer (NK) cell function (Zijlstra, M. et al. (1990) Nature 344, 742-6; Kagi, D. et al. (1994) Nature 369, 31-7). We therefore tested several other immunodeficient strains of mice for their ability to support human hematopoiesis.

Human BM and CB cells were transplanted into mice from each strain and engraftment was assessed by Southern blot analysis (Fig. 1A). Consistently high levels of engraftment were detected in NOD.SCID mice, whereas CB17.*SCID* mice showed a lower frequency and level of engraftment, as previously reported (Larochelle, A. et al. (1996) Nat Med 2, 1329-37). As expected, no engraftment was detected in immune-competent NOD recipients. Surprisingly, despite their profound immune-deficiency, engraftment failed in RAG-2, RAG-2.*Prf*^{*-*/*-*}, and RAG-2.β*₂m*^{*-*/*-*} mice on the B6 background. In contrast, NOD.*Rag-1*^{*-*/*-*} mice have been shown to support high levels of human hematopoietic cell engraftment (Shultz, L.D. et al. (2000) J Immunol 164, 2496-507). These observations suggest that additional factors other than immune-deficiency in the mouse strain background and/or host BM microenvironment dramatically impact hematopoietic engraftment in the xenotransplant setting.

### EXAMPLE 2

### Support of human long-term culture initiating cells requires NOD alleles on chromosome 2

Long-term culture initiating cells (LTC-IC) are the most primitive human hematopoietic cells that can be assayed *in vitro* (Wang, J.C.Y. et al. "Normal and leukemic human stem cells assayed in immune-deficient mice" in: Hematopoiesis - A Developmental Approach (ed. Zon, L.I.) 99-118 (Oxford University Press, New York, USA, 2001), and serve as a surrogate measure of human hematopoiesis under controlled microenvironmental conditions. LTC-IC are quantified based on their ability to generate colony-forming cells (CFC) after 5 weeks of stromal culture. To characterize strain differences in the support of human hematopoiesis, we compared the ability of BM stromal cells from NOD/Jsd (NOD) and other strains to support LTC-IC. Stromal layers from NOD mice supported human CFC production for 5 weeks of culture, whereas BM stroma from all of the other strains did not support human LTC-IC following inoculation with equivalent numbers of Lin⁻ CB cells (Fig. 1B). NOD stroma was supportive even at low input numbers of Lin⁻ CB cells (0.6 × 10⁵; Fig. 1C). We examined NOR/Lt (NOR) mice in this assay because they are a recombinant inbred strain 88% identical by descent to NOD, differing only at four Idd loci (*Idd4*, *Idd5*, *Idd9*, and *Idd13*) (Prochazka, M. et al. (1992) Diabetes 41, 98-106). In contrast to NOD, NOR cultures supported LTC-IC only when seeded with 10-fold greater numbers of Lin⁻ CB cells (Fig. 1C), and resulted in small-sized CFC. These data suggest that the unique support of primitive human hematopoiesis observed in NOD mice *in vitro* and *in vivo* is controlled by regions of genetic non-identity between NOD and NOR mice. To map the genetic loci that conferred the superior ability of the NOD strain to support human hematopoiesis, stromal cells from NOD and NOR parental mice, a series of NOD and NOR-*Idd* congenic strains, and B6.NOD-*Idd13* mice were tested in chimeric LTC (Fig. 2A). Neither the B6 or NOR parental strains were able to support development of human LTC-IC compared to NOD stroma. NOD mice congenic for NOR alleles at *Idd4*, *Idd5*, and *Idd9* displayed equivalent LTC-IC outcomes to NOD parental animals. In contrast, stromal cells isolated from NOD.NOR-*Idd13* mice (*Idd13*-BB genotype) were unable to support human LTC-IC whereas both B6 and NOR mice congenic for NOD *Idd13* (*Idd13*-NN genotype; B6.NOD-*Idd13* and NOR.NOD-*Idd13*) were supportive. Thus, the NOD-derived *Idd13* locus on chromosome 2 (Serreze, D.V. et al. (1994) J. Exp. Med. 180, 1553-1558) conferred dramatically enhanced capacity of BM stromal cells to support human LTC-IC *in vitro.*

### EXAMPLE 3

### Idd13 genotype determines support of human hematopoietic stem cell engraftment in vivo

To test whether the *Idd13* locus controls support of human hematopoietic cells capable of repopulation *in vivo* (termed SCID-repopulating cells, SRC), we generated an immune-deficient NOD congenic strain homozygous for NOR-derived *Idd13* (NOD.NOR-*Idd13-SCID*). Sublethally irradiated NOD.*SCID* and NOD.NOR-*Idd13-SCID* mice were transplanted intravenously with Lin⁻ CB cells (equivalent to 0.37-1.5 × 10⁵ CD34⁺ cells) and human CD45⁺ cell engraftment was assessed after 6-7 weeks by flow cytometry (Fig. 2B). As expected, NOD.*SCID* mice supported human engraftment over the entire CB cell dose range. In contrast, no human cell engraftment was detected in NOD.NOR-*Idd13-SCID* mice. These results confirm the *in vitro* LTC-IC data and establish that NOD alleles at the *Idd13* locus confer support of human hematopoiesis *in vivo.*

### EXAMPLE 4

### Fine mapping of candidate genes at Idd13 regulating support of human hematopoiesis

To refine the location of gene(s) at *Idd13* that regulated support of human hematopoiesis, we used a mapping strategy involving generation of *Idd13* congenic strains on both the NOD or NOR background (Fig. 3A). Stromal cells from these congenic animals were tested in chimeric LTC assays (Fig. 3A and B). Interestingly stromal cells from *Idd13* heterozygous mice (*Idd13*-BN) supported human LTC-IC, however the number of CFC generated was consistently ~50% of that seen in NOD cultures (Fig. 3A and B; third bars from left). These data were consistent with a dominant effect of NOD *Idd13* alleles on support of human LTC-IC. By repeating these analyses on eight novel *Idd13* congenic strains on both the NOD and NOR backgrounds, the support of human LTC-IC phenotype was resolved to a 960 kb region (Fig. 3A and B), defined by a single nucleotide polymorphism in *Fliz1* and the microsatellite D2Ngul1849 (Table 3). Of 15 genes in the interval, eight had functional annotation. Expression of seven of the eight genes in BM stroma and macrophages of NOD and NOR strains was assessed using real time PCR (Table 4). *Sirp*α (also called *Shps-1* and *Ptpns1*) was the only gene expressed in BM stroma that showed coding sequence polymorphism between NOD and NOR and, thus, was selected as the most promising candidate for further study.

**Table 3. Idd13 candidate interval supporting human LTC-IC.**

| Chr | Start position | Genomic marker | EnsEMBL gene ID | Gene symbol | Primer 1 | Primer 2 | SEQ ID NO. |
|---|---|---|---|---|---|---|---|
| 2 | 12867529 3 | Fliz1 | ENSMUSG 0000002738 7 | Zc3hdc8 | | | |
| 2 | 12871643 0 | | ENSMUSG 0000004285 1 | ZCC6_MOUS E | | | |
| 2 | 12874454 7 | D2Ngul172 5 | | | GCCACTACCAT CCGATTCTT | CCTGTCTTGAA AACCCAAAA | 117/118 |
| 2 | 12881500 7 | | ENSMUSG 0000002739 4 | Ttl | | | |
| 2 | 12885003 7 | | ENSMUSG 0000002739 5 | Rpo1-2 | | | |
| 2 | 12887884 0 | | ENSMUSG 0000003793 8 | Chchd5 | | | |
| 2 | 12891796 9 | | ENSMUSG 0000004340 4 | - | | | |
| 2 | 12894782 3 | | ENSMUSG 0000002739 7 | Slc20a1 | | | |
| 2 | 12896799 2 | | ENSMUSG 0000005673 8 | A730036I17Ri k | | | |
| 2 | 12901790 4 | | ENSMUSG 0000004832 7 | 2610318C08Ri k | | | |
| 2 | 12904863 3 | | ENSMUSG 0000002739 9 | Il1α | | | |
| 2 | 12911361 4 | | ENSMUSG 0000002739 8 | Il1β | | | |
| 2 | 12911892 0 | | ENSMUSG 0000005663 5 | NM_177848 | | | |
| 2 | 12920738 2 | | ENSMUSG 0000004372 7 | F830045P16Ri k | | | |
| 2 | 12934260 1 | | ENSMUSG 0000003790 2 | Sirpα**=**Ptpns1 | | | |
| 2 | 12943558 9 | | ENSMUSG 0000002740 0 | NDDB_MOUS E | | | |
| 2 | 12953089 8 | D2Jyh3941 | | | AATGACTTGTT TGCCAAGGA | TTTTATGCCCT AGGAGCCTC | 119/120 |
| 2 | 12954977 5 | | ENSMUSG 0000003788 5 | Stk35 | | | |
| 2 | 12963501 8 | D2Ngul184 9 | | | ACTCTCTGAGT AGCCTGCCCC | CCTCACCCTTA CTTGGCACC | 121/122 |

The candidate interval supporting human LTC-IC is bounded by *Fliz1* and *D2Ngul1849*, (see Fig. 3A) defining a 0.96 Mb interval on chromosome 2 containing 15 genes. A minimal candidate locus was defined by microsatellite markers *D2Ngul1725* and *D2Jyh3941* and contained 13 genes. All genomic positions and gene attributes are based on EnsEMBL version 26.33b.1. *Fliz1* primers were designed to detect a polymorphism between NOD and NOR strains in the 5' UTR.

**Table 4. Expression of annotated genes in Idd13 candidate interval.**

| **Gene symbol** | **EnsEMBL ID** | **Genotype** | **Cell type** | **Mean copy number ± SD** | **Mean copy number normalized to β-actin ± SD (× 10⁻³)** |
|---|---|---|---|---|---|
| Fliz1 | ENSMUSG00000027387 | NOR | stroma | 2180 ± 177 | 2.04 ± 0.17 |
| | | NOR.NOD- | stroma | 2107 ± 257 | 1.97 ± 0.24 |
| | | *Idd13* | macrophage | UD | UD |
| | | NOR | macrophage | UD | UD |
| | | NOR.NOD- | | | |
| | | *Idd13* | | | |
| | | | | | |
| Rpo1-2 | ENSMUSG00000027395 | NOR | stroma | | |
| | | NOR.NOD- | stroma | 9955 ± 1836 | 7.66 ± 1.41 |
| | | *Idd13* | macrophage | 4938 ± 1234 | 6.86 ± 1.7.1 |
| | | NOR | macrophage | 399 ± 23 | 0.40 ± 0.02 |
| | | NOR.NOD- | | 225 ± 39 | 0.54 ± 0.09 |
| | | *Idd1*3 | | | |
| | | | | | |
| Chchd5 | ENSMUSG00000037938 | NOR | stroma | UD | UD |
| | | NOR.NOD- | stroma | UD | UD |
| | | *Idd1*3 | macrophage | UD | UD |
| | | NOR | macrophage | UD | UD |
| | | NOR.NOD- | | | |
| | | *Idd13* | | | |
| | | | | | |
| Slc20a1 | ENSMUSG00000027397 | NOR | stroma | 13762 ± 467 | 23.80 ± 0.81 |
| | | NOR.NOD- | stroma | 19926 ± 1767 | 22.39 ± 1.99 |
| | | *Idd13* | macrophage | ND | ND |
| | | NOR | macrophage | ND | ND |
| | | NOR.NOD- | | | |
| | | *Idd13* | | | |
| | | | | | |
| Il1α | ENSMUSG00000027399 | NOR | stroma | 12104 ± 1206 | 10.09 ± 1.01 |
| | | NOR.NOD- | stroma | 11193 ± 3229 | 9.33 ± 2.69 |
| | | *Idd13* | macrophage | 2321338 ± | 1304.12 ± 320.79 |
| | | NOR | macrophage | 571008 | 1198.81 ± 53.24 |
| | | NOR.NOD- | | 4303725 ± | |
| | | *Idd13* | | 191113 | |
| | | | | | |
| Il1β | ENSMUSG00000027398 | NOR | stroma | 19813 ± 1701 | 17.08 ± 1.40 |
| | | NOR.NOD- | stroma | 12324 ± 3185 | 15.40 ± 3.90 |
| | | *Idd13* | macrophage | 9167234 ± | 7051.72 ± 94.46 |
| | | NOR | macrophage | 122794 | 4595.18 ± 320.08 |
| | | NOR.NOD- | | 8730824 ± | |
| | | *Idd13* | | 608149 | |
| | | | | | |
| Stk35 | ENSMUSG00000037885 | NOR | stroma | 221 ± 16 | 0.44 ± 0.44 |
| | | NOR.NOD- | stroma | 400 ± 15 | 0.57 ± 0.02 |
| | | *Idd13* | macrophage | ND | ND |
| | | NOR | macrophage | ND | ND |
| | | NOR.NOD- | | | |
| | | *Idd13* | | | |
| | | | | | |

| | | | | | |
|---|---|---|---|---|---|
| UD = below limit of detection ND= not done See Materials and Methods for description of real time PCR | | | | | |

### EXAMPLE 5

### Strain-dependent sequence and biochemical variation in Idd13 candidate gene Sirpα

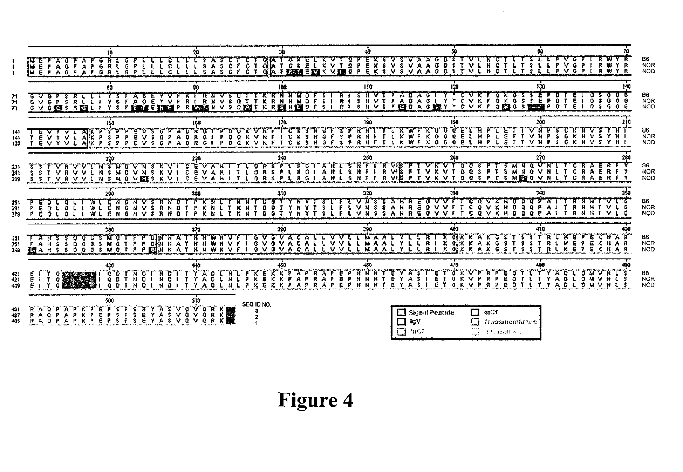

Signal regulatory protein α (SIRPα) is an Ig-superfamily transmembrane protein with intracellular docking sites for two Src homology domain-containing protein tyrosine phosphatases SHP-1 and SHP-2 (Barclay, A.N. & Brown, M.H. (2006) Nat Rev Immunol 6, 457-64). *Sirp*α is abundantly expressed in BM stroma and hematopoietic cells (data not shown). We used PCR amplification and bi-directional sequencing of cDNA from BM-derived macrophages to identify variations in the coding regions of NOD, NOR and BALB/c mice. The NOR sequence is identical to B6 with the exception of 4 additional amino acids in the B6-derived cytoplasmic domain (Fig. 4) that likely reflects variation between two splice donor sites in exon 7 (Sano, S. et al. (1999) Biochem J 344 Pt 3, 667-75). Comparison of the *Sirp*α coding sequence between NOD and NOR revealed 24 amino acid differences, 20 of these in the extracellular IgV-like domain of molecule where the NOD sequence displays 18 substitutions and two deletions compared to NOR and B6 (Fig. 4 and Fig. 8A). This observed variation in the *Sirp*α the N-terminal IgV-like domain between NOD and NOR or B6 is more extensive than that previously reported in this region amongst the B6, BALB/c and 129/Sv strains (Sano, S. et al. (1999) Biochem J 344 Pt 3, 667-75).

To assess the consequences of SIRPα sequence variation between NOD and NOR mice, protein lysates were prepared from BM-derived macrophages, and SIRPα expression was examined by immunoblotting with polyclonal antibodies directed against the cytoplasmic domain which is sequence identical in NOD and NOR mice (Fig. 4). No quantitative strain differences in SIRPα expression were detected under the conditions examined. Electrophoretic mobility of the NOD protein was slightly less than that of the NOR protein (Fig. 5A). SIRPα is glycosylated (van den Nieuwenhof, I.M. et al. (2001) J Cell Sci 114, 1321-9), so we examined the possibility that strain differences in the apparent molecular weight reflected differential glycosylation. Protein lysates from BM-derived macrophages were treated with N-glycosidase F and examined by immunoblotting (Fig. 5B). Following removal of N-linked glycans, SIRPα from both strains migrated faster, with roughly equal mobility. These results suggest that the extensive polymorphism we detected in the extracellular portion of the NOD and NOR proteins results in differential glycosylation. Thus, the amino acid sequence and post-translational modifications dissimilarities between NOD and NOR strains are consistent with *Sirp*α being the *Idd13* gene conferring strain-specific differences in human hematopoietic support.

### EXAMPLE 6

### Murine macrophages display strain-specific variation in support of human LTC-IC

Murine SIRPα is most abundantly expressed in neurons and myeloid cells (Veillette, A. et al. (1998) J Biol Chem 273, 22719-28) with evidence of differentially glycosylated forms in heart, liver, kidney and other tissues (Sano, S. et al. (1999) Biochem J 344 Pt 3, 667-75). SIRPα ligation regulates multiple macrophage functions including inhibition of phagocytosis (Oldenborg, P.A. et al. (2001) J Exp Med 193, 855-62; Blazar, B.R. et al. (2001) J Exp Med 194, 541-9) and TNFα production (Smith, R.E. et al. (2003) Blood 102, 2532-40), and increasing nitric oxide production (Alblas, J. et al. (2005) Mol Cell Biol 25, 7181-92). We and others have shown that a human CD122⁺ cell subset distinct from NK cells, likely macrophages, may play a role in host resistance to human hematopoietic engraftment in the xenotransplantation setting (McKenzie, J.L. et al. (2005) Blood 106, 1259-61; Shultz, L.D. et al. (2003) Exp Hematol 31, 551-8). In addition, macrophages are a component of hematopoietically supportive BM stromal cultures (Hauser, S.P. et al. (1995) J Histochem Cytochem 43, 371-9). Based on these observations, we tested whether macrophages conferred the strain-specific differences in human hematopoietic support we observed in chimeric LTC-IC assays. Peritoneal macrophages from NOD, NOR, and NOR congenic mice heterozygous at the *Idd13* locus (NOR.BN-*Idd13*) were harvested and added to human LTC-IC cultures employing MS-5 stromal cells, a murine cell line that supports human hematopoiesis (Issaad, C. et al. (1993) Blood 81, 2916-24). Murine macrophages were seeded onto established MS-5 cultures at doses of 200, 2,000 and 20,000 cells per well, followed 24 hours later by addition of 2,000 Lin⁻ CB cells. Macrophages of all strains had a dose-dependent effect on the number of CFC generated after 3 to 4 weeks in LTC (Fig. 6A). NOR macrophages exerted greater suppression of human LTC at all cell doses than NOD macrophages; at 2,000 and 20,000 macrophages/well, the strain difference was significant (p<0.008). NOR.BN-*Idd13* had an intermediate effect between NOR and NOD macrophages consistent with our previous observations of a (co)-dominant effect of *Sirp*α alleles on support of human hematopoiesis. Replicate experiments performed with BM-derived macrophages showed similar results (data not shown). These findings demonstrate a *Idd13* genotype-specific, dose-dependent suppressive effect of murine macrophages on human hematopoiesis *in vitro.*

### EXAMPLE 7

### Sirpα confers differential capacity of NOD macrophages to support human hematopoiesis

To directly test whether the NOD *Sirp*α variant confers the observed strain-specific effects of macrophage-mediated suppression of human hematopoiesis, we constructed lentivirus vectors expressing NOD-derived *Sirp*α plus *Gfp* (SIRP), or *Gfp* alone (CEP). Infection of Jurkat cells or NOR BM-derived macrophages with SIRP resulted in high level expression of SIRPα protein, which in macrophages exhibited NOD-type mobility on Western blot analysis suggesting that it was appropriately glycosylated in these cells (Fig. 6B) To assess the effect of *Sirp*α in chimeric LTC, NOR BM-derived macrophages were infected with SIRP or control CEP lentivirus prior to seeding on MS-5 stromal cultures. Human Lin⁻ CB cells were added the next day and generation of CFC was assessed 25 to 31 days later. Cell dose effects were again evident in this setting where seeding 20,000 macrophages/well suppressed human hematopoiesis regardless of *Sirp*α genotype. However, expression of NOD-derived SIRPα by NOR macrophages resulted in substantially greater human CFC support compared to either non-manipulated or CEP-infected NOR macrophages (Fig. 6C and data not shown). Results from these gain-of-function experiments confirm that SIRPα confers strain-specific, macrophage-mediated effects in chimeric LTC, providing direct support that this gene is responsible for the *Idd13* locus effect on the support of human hematopoesis both *in vitro* and *in vivo.*

### EXAMPLE 8

### Allelic variation in murine Sirpαconfers differential binding to human CD47

The cellular ligand of SIRPα is the ubiquitously expressed integrin-associated protein CD47, also a member of the Ig superfamily (Seiffert, M. et al. (1999) Blood 94, 3633-43). CD47 binds to the extracellular IgV-like domain of SIRPα (Seiffert, M. et al. (2001) Blood 97, 2741-9; Vernon-Wilson, E.F. et al. (2000) Eur J Immunol 30, 2130-7). Previous studies have shown that human SIRPα displays cross-species binding to porcine CD47, but not to CD47 from mouse (B6), rat or cow (Subramanian, S. et al. (2006) Blood 107, 2548-56). Having identified 20 amino acid differences in the IgV-like domain between the NOD and NOR alleles of SIRPα (Fig. 4), we asked whether these variations conferred differential binding of murine SIRPα to human CD47. BM-derived macrophages from NOD and NOD.NOR-*Idd13* mice had similar proportions of CD11b⁺ cells (Fig. 7A), and these cells expressed equivalent amounts of SIRPα (Fig. 7B and C). However, the human CD47 extracellular domain fused to human IgG1 Fc (hCD47-Fc) (Latour, S. et al. (2001) J Immunol 167, 2547-54) showed far greater binding to NOD compared to NOD.NOR-*Idd13* macrophages (Fig. 7B and D), demonstrating *Idd13*-genotype-dependent interaction between murine SIRPα and human CD47. NOD, NOR and NOD.NOR-*Idd13* macrophage extracts contained equal amounts of SIRPα, but immunoprecipitation of SIRPα by hCD47-Fc was neglible from both NOR and NOD.NOR-*Idd13* lysates, in contrast to the efficient recovery from NOD lysates (Fig. 7E). Taken together, these data indicate that NOD-derived SIRPα displays far greater reactivity with human CD47 than does the NOR (or B6)-derived protein, providing a molecular mechanism for the enhanced support of human hematopoesis in NOD.*SCID* mice.

### EXAMPLE 9

### Protein sequence alignments of murine and human SIRPαIgV domains and Sirpα polymorphisms within human populations

Above, we have used a positional genetics approach combined with functional assays of hematopoiesis to identify a *Sirp*α as an important regulator of interactions between human stem and progenitor cells and the BM microenvironment *in vitro* and *in vivo.* We show that expression on murine macrophages of a SIRPα protein variant capable of binding human CD47 is both necessary and sufficient for support of human hematopoiesis in chimeric LTC. These findings implicate macrophages as important mediators of engraftment following hematopoietic stem cell transplantation.

The superior human hematopoietic engraftment achieved in NOD.*SCID* mice compared to other immune-deficient mouse strains suggests that additional host strain-specific factors affect hematopoietic engraftment. We found a robust genetic association between the *Idd13* locus in murine macrophages and support of human hematopoiesis in chimeric cultures and *in vivo.* Direct gain-of-function studies using lentiviral gene transfer confirm the *in vitro* LTC-IC data and implicate *Sirp*α as the gene responsible for the *Idd13* locus effect. In the hematopoietic system, SIRPα is primarily expressed on myeloid cells and contains cytoplasmic immunoreceptor tyrosine-based inhibition motifs (ITIM) which mediate inhibitory signals leading to reduced phagocytosis by macrophages, inhibition of neutrophil migration, and attenuated production of the inflammatory cytokine TNFα (reviewed in Barclay, A.N. & Brown, M.H. (2006) Nat Rev Immunol 6, 457-64). CD47, the only known cellular ligand of SIRPα, is ubiquitously expressed and modulates multiple cellular actions on hematopoietic cells including platelet activation, migration, and adhesion, leukocyte adhesion and cytokine production, and T cell responsiveness (reviewed in Brown, E.J. & Frazier, W.A. (2001) Trends Cell Biol 11, 130-5). Both SIRPα and CD47 are immunoglobulin superfamily members and their interaction is mediated through their respective IgV-like domains (Seiffert, M. et al. (2001) Blood 97, 2741-9). We identified 20 amino acid differences in the IgV-like domain between NOD and NOR SIRPα, as well as dissimilar glycosylation between the two proteins, suggesting that differential interaction with human CD47 could underlie the strong effect of *Sirp*α variation on support of human hematopoiesis *in vitro* and *in vivo.* This mechanism is supported by our biochemical and flow cytometric analyses demonstrating enhanced binding to human CD47 by the NOD-derived SIRPα compared to the NOR-derived protein.

CD47-SIRPα interaction has been implicated in regulation of phagocytosis-mediated clearance of red blood cells (Oldenborg, P.A. et al. (2000) Science 288, 2051-4) and leukocytes (Gardai, S.J. et al. (2005) Cell 123, 321-34), wherein expression of CD47 on the hematopoietic cells serves as a "marker of self", inhibiting phagocytosis by macrophages expressing SIRPα. A similar mechanism was suggested to modulate phagocytosis of a porcine lymphoblastoid cell line (LCL) by human macrophages recovered from liver allografts, based on *in vitro* observations that phagocytosis was attenuated by pre-incubation of the human macrophages with human CD47-Fc, or by ectopic expression of human CD47 on LCL transfectants (Ide, K. et al. (2007) Proc Natl Acad Sci U S A 104, 5062-6). Our evidence extends these prior observations by demonstrating that genetic variation in the *Sirp*α IgV domain impacts the degree of CD47 interaction and can exert an "all or nothing" effect on engraftment of human HSC, linking the previous findings to an *in vivo* transplantation setting in which outcome is modulated by host macrophages. Importantly, without being bound to any theory, macrophage-mediated phagocytosis may not be the sole or primary effector mechanism underlying hematopoietic support *in vitro* or *in vivo,* as we observed a gradual disappearance of human hematopoietic cells in NOR chimeric cultures (data not shown), an observation not readily consistent with rapid phagocytosis-based clearance. Moreover, macrophages from all mouse strains displayed a dose-dependent suppressive effect on the growth of human LTC-ICs, suggesting that SIRPα signals inhibit macrophage secretion of inflammatory cytokines such as TNFα, as previously shown for macrophages stimulated by pathogen products (Smith, R.E. et al. (2003) Blood 102, 2532-40) . In addition to SIRPα effects on macrophages, SIRPα-CD47 binding may activate CD47-induced signalling pathways within the human hematopoietic cells. Thus, any number of the pleiotropic effects of SIRPα-CD47 interactions may influence human HSC survival and engraftment.

Our observations in the xenotransplantation setting may have broader implications for clinical HSC transplantation. There is a large body of evidence demonstrating that the observed behavior of primitive hematopoietic stem/progenitor cells transplanted into NOD.SCID mice is predictive of the clinical setting (Wang, J.C.Y. et al. "Normal and leukemic human stem cells assayed in immune-deficient mice" in: Hematopoiesis - A Developmental Approach (ed. Zon, L.I.) 99-118 (Oxford University Press, New York, USA, 2001).

To further explore whether the functional variations we have observed in the murine system are relevant in humans, we investigated *SIRP*α polymorphism in human populations. We sequenced the *SIRP*α IgV domain from 37 unrelated normal Caucasian (CEU), African (YRI), Chinese (CHB) and Japanese (JPT) individuals from the human HapMap genome project (Table 5) and identified 10 distinct *SIRPα* IgV alleles reflecting combinatorial variation at 18 amino acids (Fig. 8b). A recently reported high-resolution X-ray crystal structure of the *SIRP*α IgV domain predicts the critical residues that contribute to the CD47 binding surface (Subramanian, S. et al. (2006) Blood Cells Mol Dis 36, 364-72). Strikingly, we observed human allelic variations at predicted CD47 binding residues (Fig. 8b). Moreover, we observed human allelic variations clustered in the same sub-regions of the *SIRP*α IgV domain that distinguish NOD from NOR alleles (as well as other mouse strains; Fig. 4 and Fig. 8b). Human polymorphism was also seen in the orthologous position to a variation between NOD and NOR (amino acid 72 serine v. asparagine) which may contribute to the distinct patterns of SIRPα N-linked carbohydrate addition we observed in these mouse strains (Fig. 5b and Fig. 8b). Collectively, these data show that, as in laboratory mice, human *SIRP*α is highly polymorphic in the IgV domain, and suggest that allelic variants will impact posttranslational processing and CD47 ligand interaction.

Our findings identify a new axis of genetically regulated HSC engraftment. Classic graft rejection is an immunologic phenomenon mediated by residual host T cells or NK cells, and rejection rate is related to the degree of HLA disparity between recipient and donor. We show that in addition to T and NK cells, host macrophages play an important role in engraftment mediated through SIRPα-CD47 interactions. Moreover, our findings may be relevant for BM failure syndromes with possible immune-mediated pathogenesis.

It is shown that the effect of mouse macrophages on support of human HSC depends upon SIRPα alleles (is sequence-dependent), and on the number of mouse macrophages in the LTC-IC assays. At high input cell numbers even NOD-derived macrophages reduced support for human HSC in these assays. In clinical settings where the number of human HSC transplanted is limiting and the number of recipient SIRPα bearing macrophages is large, enhanced engraftment could be achieved by treating the recipient with therapeutic doses of CD47-Fc or an equivalent soluble reagent capable of efficient and specific triggering of SIRPα inhibitory signals in recipient macrophages. Once the HSC and their lineage committed progeny engraft and repopulate the recipient, the need for therapeutic engagement of SIRPα might decrease as the established graft produces large numbers of CD47+ cells producing a favorable ratio relative to the remaining recipient-derived macrophages.

A person skilled in the art would understand that one could continue to analyze the degree of human polymorphism in SIRPα by sequencing normal, unrelated individuals, for example, through the available human HapMap samples obtainable through the Sick Kids Centre for Applied Genomics (Toronto, Canada). For example, a 1-2microgram DNA sample from donor and recipient archival blood samples in the HLA lab would be used for sequencing SIRPα using standard sequencing facilities. The donor/recipient frozen blood sample pairs would be given a unique anonymous ID lacking any diagnosis or any outcome details. Particular attention would be paid to sequence exon 3 of this gene which encodes the IgV domain that is critical to ligand binding and function, and also to sequence two additional exons that encode the intracellular regions of the gene which include important amino acid residue for conveyance of signals emanating from SIRPα. One would create a SIRPα sequence database of these individuals, stripped of any patient identifiers, and perform statistical analysis for association between SIRPα variations and bone marrow engraftment outcomes in the donor-host pairs.

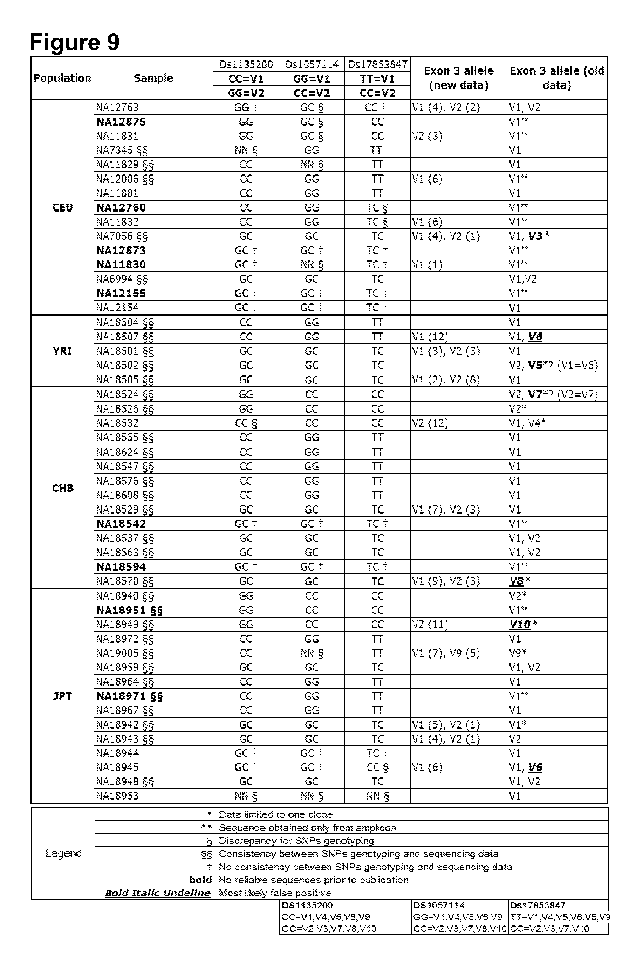

Referring to Figure 9, three SNP assays (given Ds# notations) were established to detect variable nucleotides underlying several non-synonymous coding polymorphisms we detected among the human HapMap samples. Polymerase chain reaction (PCR) amplification primers and detection probes were designed based on Applied Biosystems TaqMan system (www.appliedbiosystems.com). The discrimination between SIRPα variants (V1, V2... etc) is shown at bottom of the Table in Figure 9. Figure 9 shows the validation of the discrimination power of each SNP assay by independent sequencing of the SIRPα IgV domain PCR amplified from each of the human HapMap samples shown at the left side of the table. The extent of normal human polymorphism in SIRPα IgV domain sequence is to be determined and use of both SNP assays and sequencing for use in clinical studies is to be optimized. The methods and data employed are widely accessible and known by those of ordinary skill in the art, see for example the International HapMap Project (www.hapmap.org).

### EXAMPLE 10

### Production of hCD47-Fc Protein

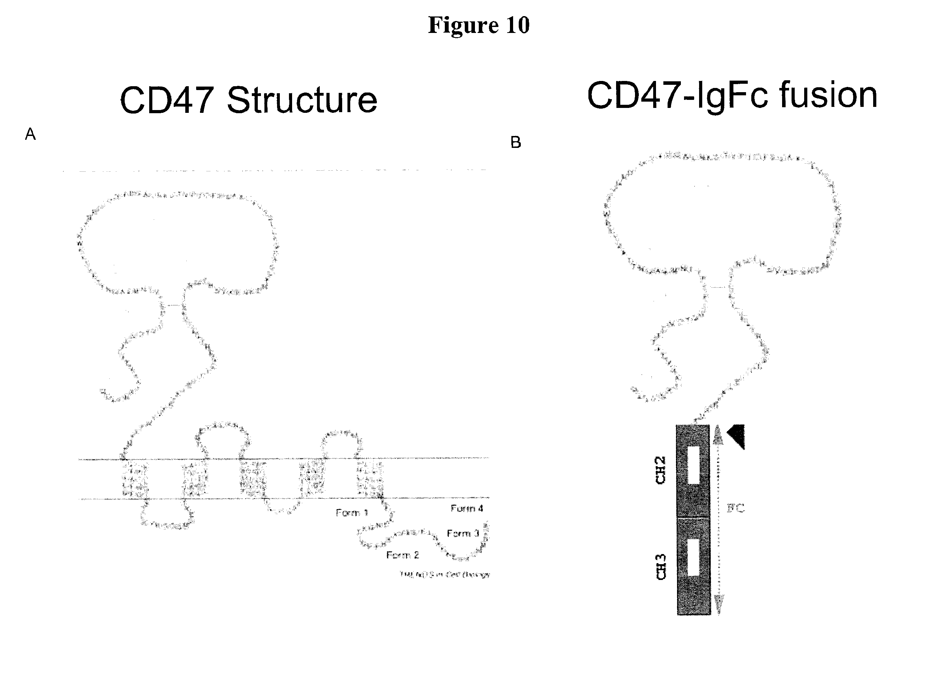

Figure 10a is a graphic depiction of the CD47 protein including extracellular IgV-like loop, five membrane spanning regions and short cytoplasmic tail. Figure 10b depicts the CD47-Fc fusion protein representing the CD47 IgV-like domain fused to a human Ig Fc region.

CD47-Fc fusion proteins were prepared as described in the above methods and materials.

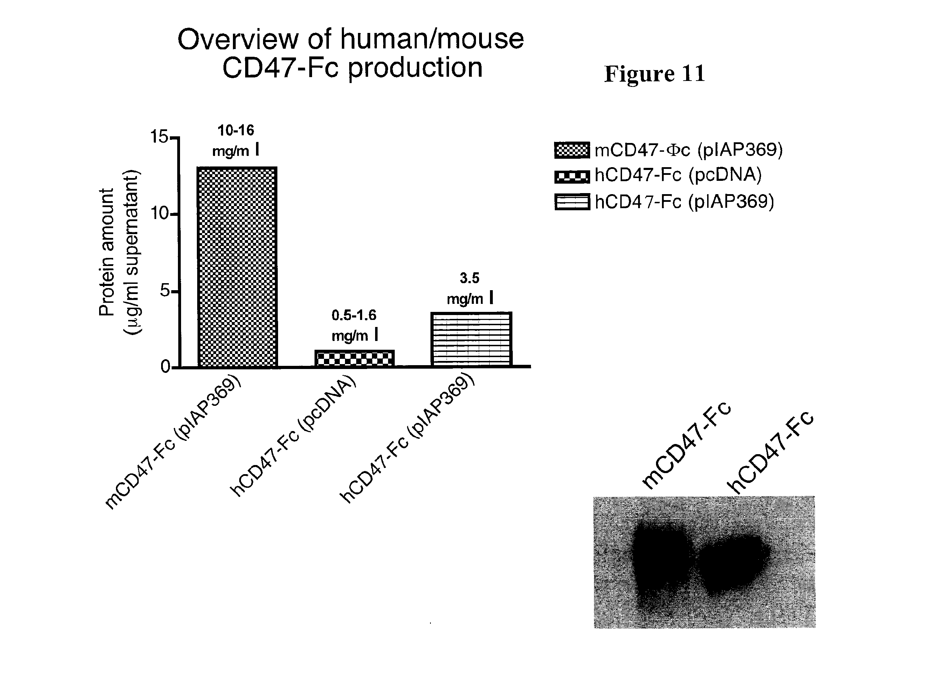

Figure 11a shows a histogram depicting protein production in the supernatant of cultured cells transfected with mCD47-Fc and two hCD47-Fc constructs prepared with different plasmid backbones (pcDNA and pIAP369). Figure 11b is an immunoblot of an SDS-PAGE depicting the fusion proteins following purification from culture supernatant. Neither hCD47-Fc construct produced as well as the mCD47-Fc construct prompting further optimization.

### EXAMPLE 11

### Optimization of hCD47-Fc Protein Production

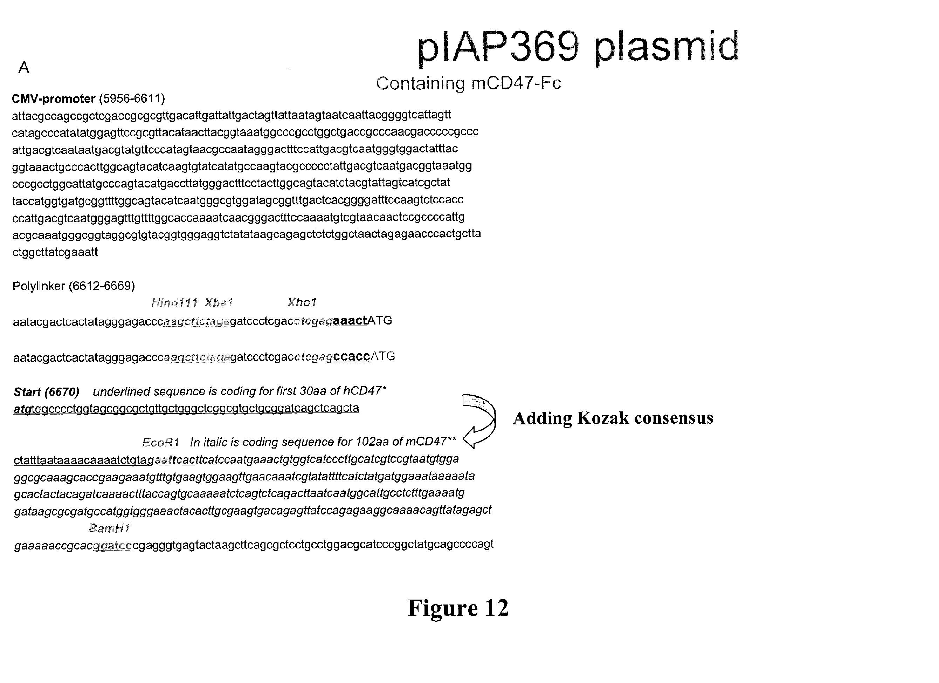

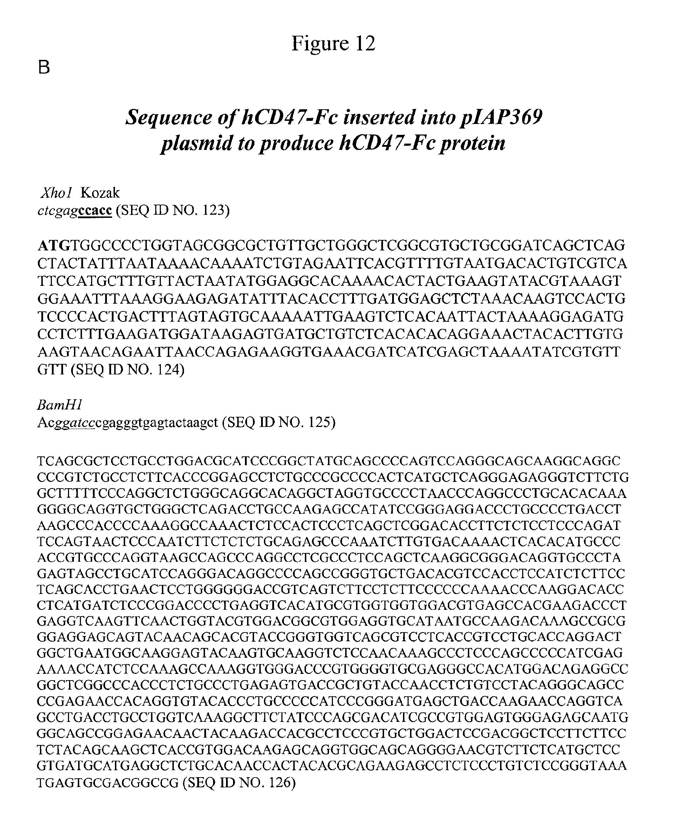

Applicants introduced a consensus initiation site to enhance protein production. Referring to Figure 12a, the pIAP369 plasmid construct containing mCD47-Fc was used as a scaffold to introduce a eukaryotic translation initiation site (Kozak, M Nucleic Acids Res. 1984, 12(2):857-72) called a Kozak sequence. The resulting plasmid was then digested with restriction enzymes to remove the *mCD47* domain which was replaced with the *hCD47* IgV-like domain sequence by ligation and re-cloning in bacteria. The resulting plasmid was sequenced to ensure in-frame introduction of the hCD47 sequence and verification of the Kozak sequence. The foregoing utilized typical molecular cloning techniques as would be known to a person of ordinary skill in the art. Figure 12b shows the sequence hCD47-Fc inserted into the pIAP369 plasmid, showing the Kozak consensus (SEQ ID NO.123), hCD47 fragment (SEQ ID NO.124), linker (SEQ ID NO.125) and Fc (SEQ ID NO.126).

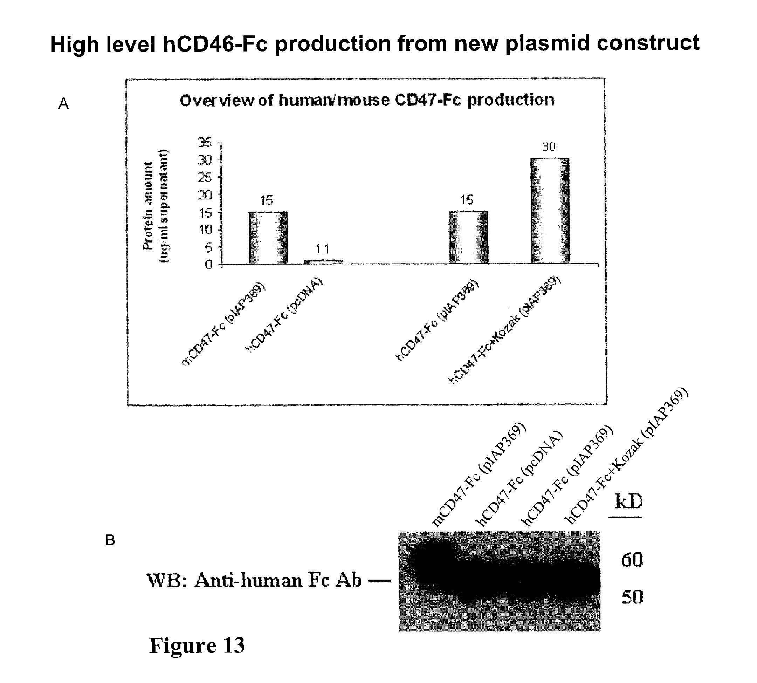

Figure 13a shows the production of mouse and human CD47-Fc assayed in the supernatant of cells transfected with one of four different constructs prepared as above. The data show that introduction of the eukaryotic initiation (Kozak) sequence enhanced production of hCD47-Fc (compare two right hand columns) to 30mg/L of supernatant. Figure 13b shows immunoblot displaying an anti-human Fc antibody reacting with the protein G-purified CD47-Fc proteins. Note that differential post-translational additional of carbohydrate moieties produces differences in SDS-PAGE mobilities for mouse and human CD47-Fc.

Applicant have succeeded in producing significant amounts of highly purified CD47-Fc fusion proteins suitable for *in vivo* testing in the NOD.*SCID* xenotransplant model.

### EXAMPLE 12

### Mouse CD47-Fc Binding to NOD and NOR SIRPα

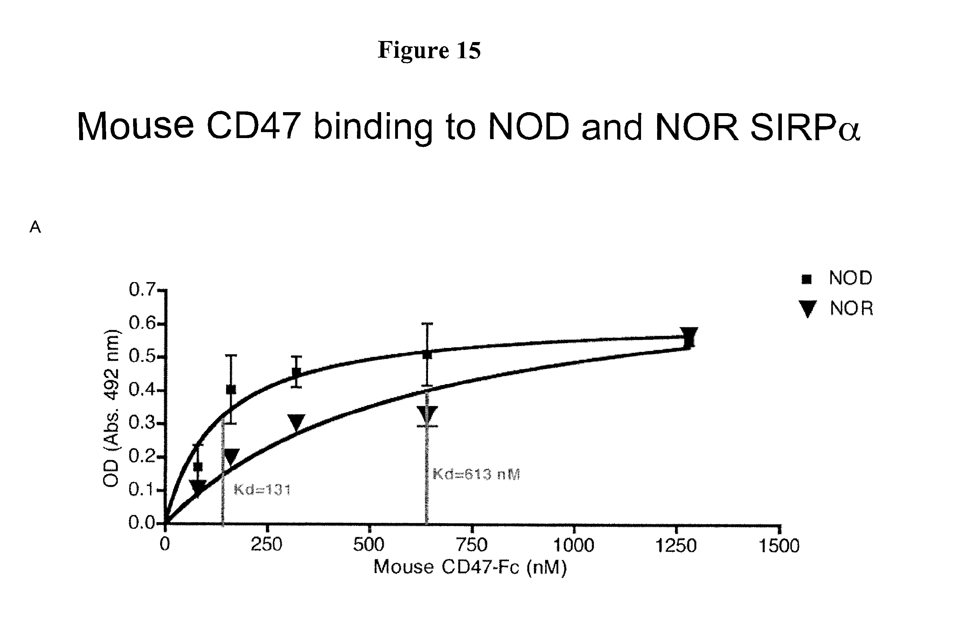

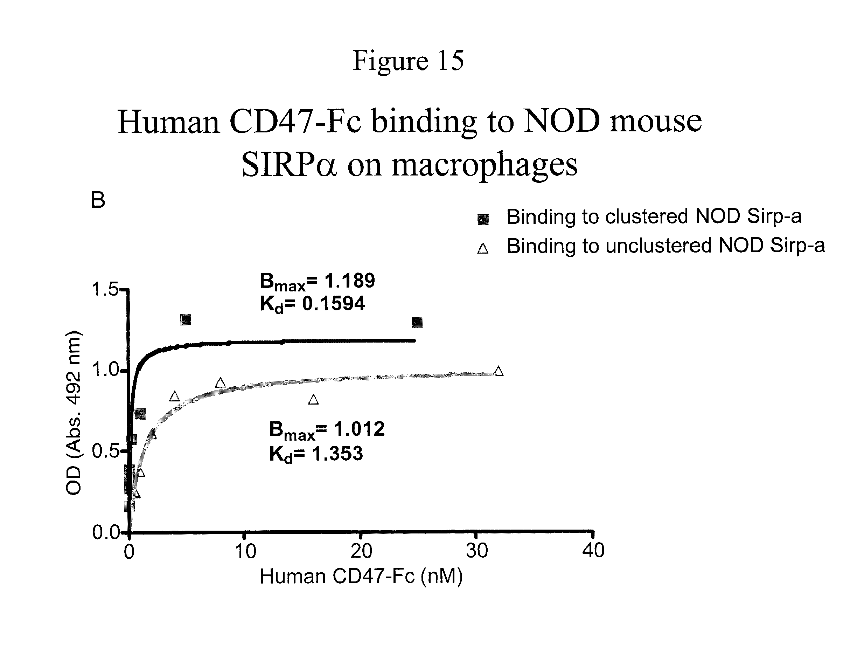

Assay of mCD47-Fc binding to SIRPα on the surface of freshly prepared NOD and NOR macrophages was determined using the assay described in the materials and methods section in relation to Figure 14. Figure 15 shows the results that the Kd of NOD SIRPα-mCD47 binding is with a greater than 4 fold (about 4.7 fold) less than the Kd of NOR SIRPα-mCD47 interaction representing a greater than 4 fold higher affinity of interaction by the NOD compared to the NOR variant of SIRPα. These data suggest that the SIRPα variations we have observed in human SIRPα IgV domain sequence is predicted to confer significant differences in binding to human CD47 which may affect the efficacy of HSC transplantation.

### EXAMPLE 13

### Human CD47-Fc Binding to NOD and NOR SIRPα

Assay of hCD47-Fc binding to SIRPα on the surface of freshly prepared NOD and NOR macrophages was determined using the assay described in the materials and methods section in relation to Figure 14. Two conditions were tested: first, binding to SIRPα on unmanipulated fresh macrophages ("unclustered; triangles) and second, to macrophages that had been pre-treated with a monoclonal antibody to SIRPα ectodomain ("clustered"; squares). The latter condition was examined to mimic the effect of engaging CD47 displayed on the surface of human HSC and their progeny cells. In that setting, the CD47 binding would create a higher valency, "clustering" the bound SIRPα proteins on the mouse macrophage. In Figure 15b, the results show that the Kd of NOD SIRPα-hCD47 binding is 1.6 nM, representing a high affinity interaction. Strikingly, under the clustering conditions, the affinity rise by more than 10-fold providing a Kd of 0.16nM. These data suggest that the human CD47 binding to NOD-derived SIRPα IgV domain is very high and predictive of a strong therapeutic effect of the CD47-Fc fusion proteins *in vivo.*

### EXAMPLE 14

### Evaluation of the Role of SIRPα-Mediated Signal Transduction on Engraftment of HSC

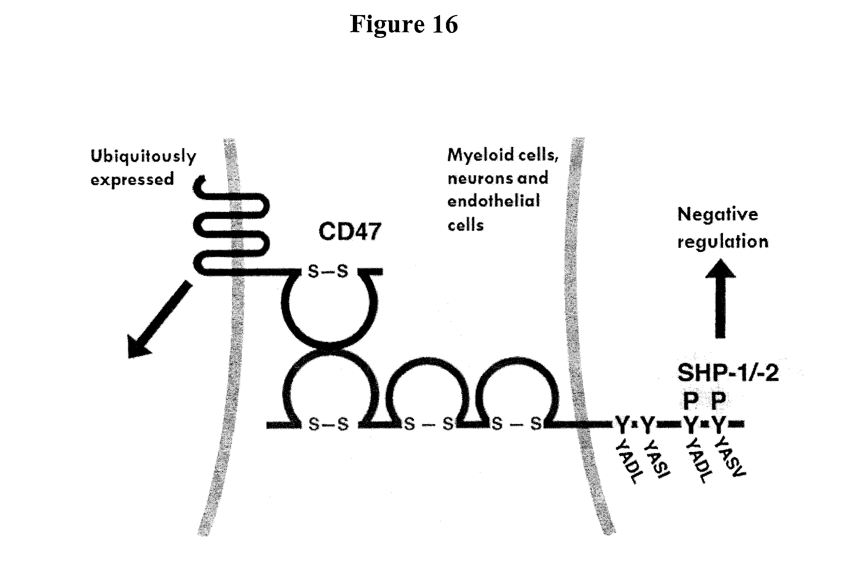

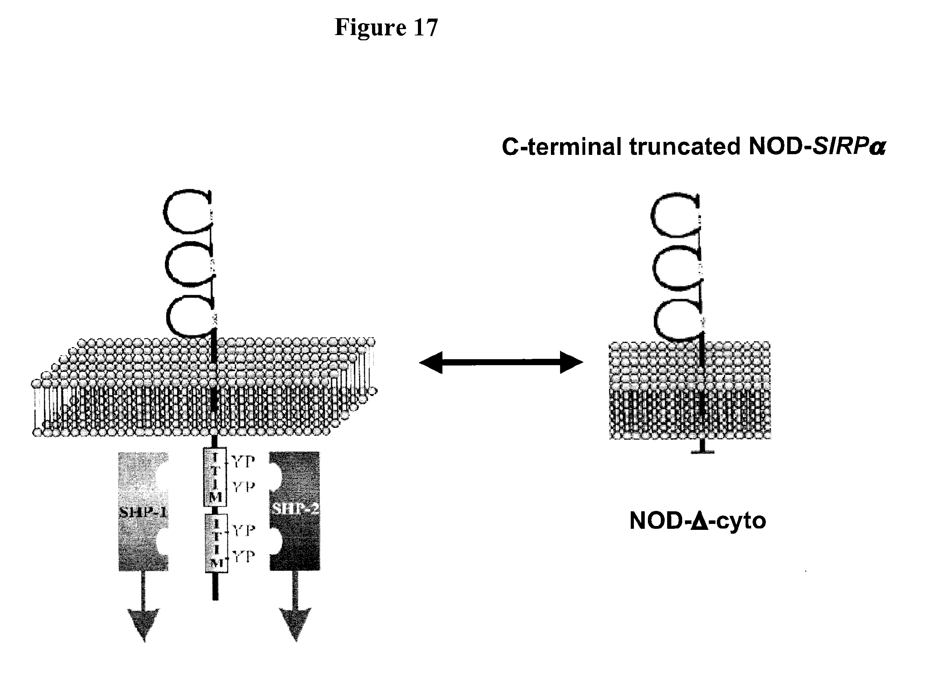

Other studies suggest that SIRPα-CD47 binding elicits bi-directional signals through each of the proteins (represented by the schematic shown in Figure 16). Applicants evaluated whether signals through SIRPα, mediated by CD47 binding, are required for HSC engraftment. Figure 17 shows preparation of a truncated version of the human HSC "permissive" NOD version of SIRPα ("NOD-Δ-cyto") (left side) incapable of transmitting signals by intentional deletion of the two immuno tyrosine inhibitory motifs (ITIM) required to link to critical downstream phosphatases SHP-1 and SHP-2 (right side).

A long-term culture initiating cell (LTC-IC) assay (performed using method described in Takenaka et al Nature Immunol 2007 Dec, 8(12): 1313-23) to determine requirement for SIRPα-CD47 mediated signaling in human HSC survival.

This experiment compares the capacity of full length cytoplasmic truncated NOD SIRPα to support human HSC survival in LTC-IC assays. Five conditions were tested using graded doses of mouse macrophages of either NOD or NOR origin transduced with different lentiviruses:
1) NOR macrophages infected with an "empty" lentivirus (NOR-CEP "diamonds")
2) NOD macrophages not infected with virus (NOD uninf; filled triangles)
3) NOD macrophages infected with "empty" lentivirus (NOD-CEP; circles)
4) NOR macrophages infected with lentivirus containing full length NOD SIRPα (NOR-SIRP; squares)
5) NOR macrophages infected with lentivirus containing truncated NOD SIRPα (NOD-Δ-cyto; open triangles)

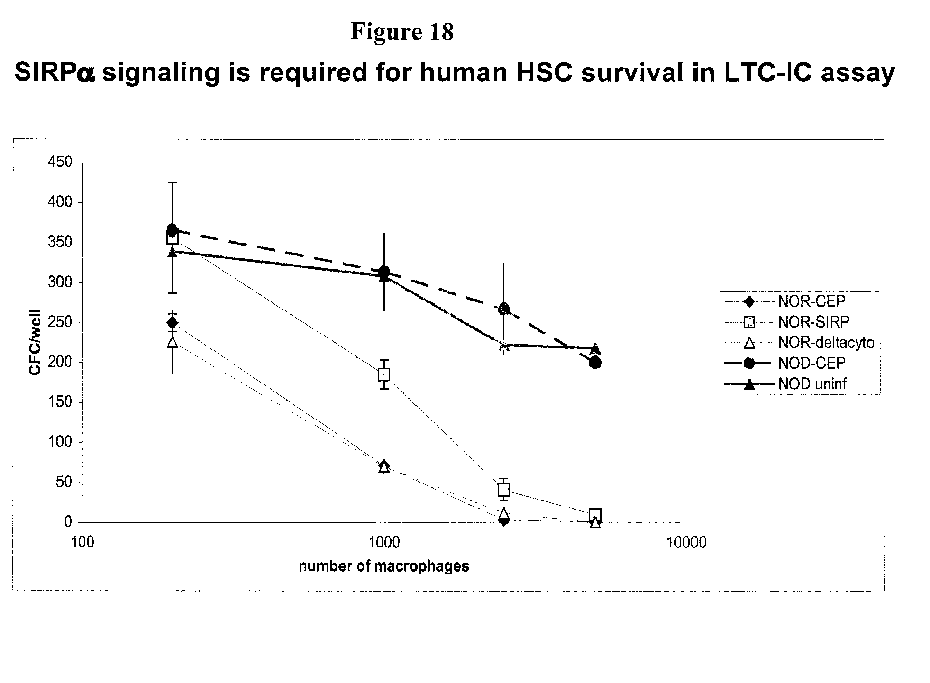

Referring to Figure 18, infection of NOR macrophages with lentivirus containing a full length NOD SIRPα construct significantly enhances human HSC survival compared to NOR macrophages infected with the empty virus (squares v. diamonds). In contrast, infection of NOR macrophages with a lentivirus containing the cytoplasmic truncated version of NOD SIRPα is not distinguishable from infection of NOR macrophages with empty virus (diamonds v. open triangles). These data show that support of human HSC depends upon CD47-dependent signaling through SIRPα. Therefore, therapeutics development to enhance human HSC transplantation, survival and differentiation in patients should focus on manipulation of signals through SIRPα.

Applicants reasoned that the NOD.*SCID* xenotransplantation data reported in Takenaka et al (*supra*) emulate clinical settings where the patient receiving a small population of HSC has abundant macrophages expressing SIRPα. In this "non-myeloablative" setting, binding affinity between CD47 on the donor HSC and SIRPα expressed on the patient's macrophages could control the efficiency of triggering SIRPα-mediated signals and could determine whether the HSC persisted or eliminated.

### EXAMPLE 15

### Evaluation in vivo of hCD47-Fc Therapy in Human HSC Engraftment

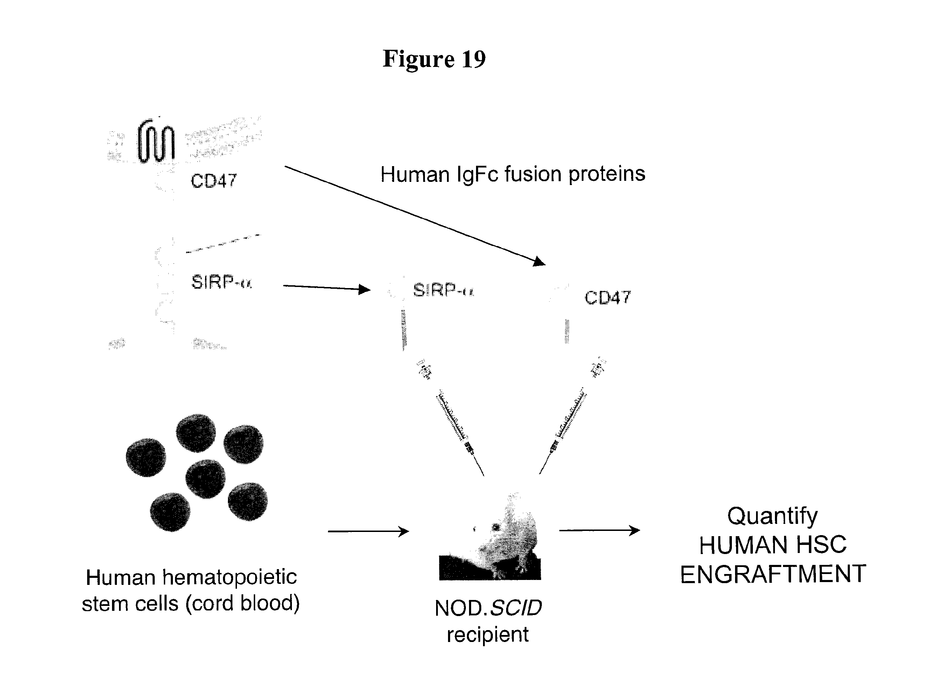

Figure 19 is a general outline of a study designed to evaluate *in vivo* the effect of CD47 fusion protein on HSC engraftment. The hCD47-Fc fusion protein was injected into NOD.*SCID* hosts together with introduction of graded doses of human newborn cord blood hematopoietic stem cells. Human HSC were prepared by removal of mature lineage-committed cells using a cocktail of monoclonal antibodies and immunomagnetic beads as previously described (J. L. McKenzie et al., Nat Immunol. 2006, 11:1225-33). HSC engraftment is followed by flow cytometric examination with human-specific antibodies to cell surface markers (CD34, CD45) in blood, spleen and bone marrow of the recipient mice.

### Human CD47-Fc Fusion Protein Confers Enhanced Engraftment of Human HSC

A study performed was to determine whether treatment with human CD47-Fc fusion protein would influence engraftment of human cord blood stem cells upon transplantation into NOD.SCID mice. Human newborn cord blood cells were depleted with a cocktail of antibodies against blood cell lineage markers using our previously published methods (Vormoor J, Lapidot T, Pflumio F, Risdon G, Patterson B, Broxmeyer HE, Dick JE. Blood. 1994, 83(9):2489-97) resulting in an enriched populations of HSC. Cohorts of age matched female NOD.SCID mice were preconditioned with low dose irradiation as we previously described (Vormoor J, Lapidot T, Pflumio F, Risdon G, Patterson B, Broxmeyer HE, Dick JE. Blood. 1994, 83(9):2489-97). Twenty to forty thousand lineage depleted CB cells were injected into NOD.SCID mice that were injected intraperitoneally with either purified human CD47-Fc protein or an irrelevant human Fc isotype control preparation. The treatment was administered three hours before injection of the CB cells, and then 3 times/week for 3 weeks (7.5-30 micrograms/dose). At the conclusion of the experiment, all mice were sacrificed and bone marrow cells prepared from the tibia and femur. The bone marrow cells were stained with a series of monoclonal antibodies and analyzed by flow cytometry. Cells staining with anti-human CD45 were quantified to determine the extent of human HSC engraftment.

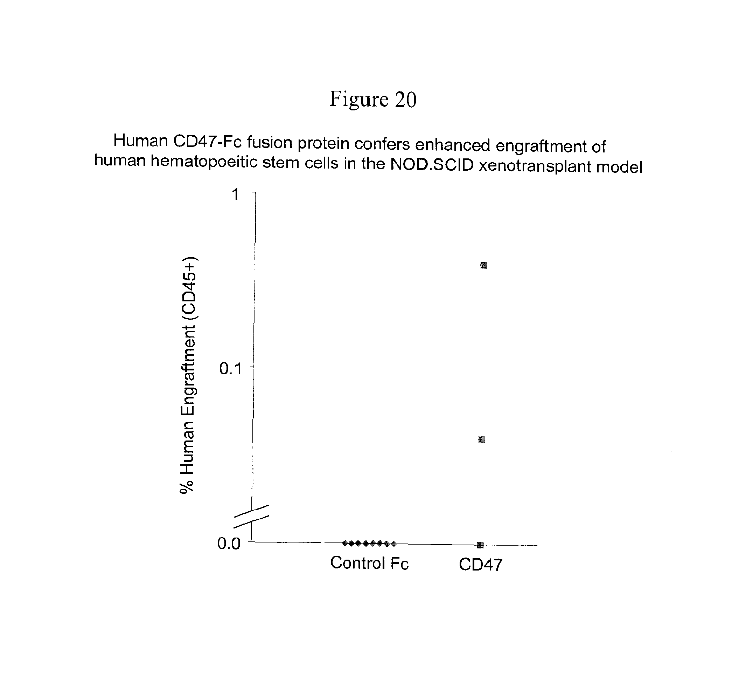

Referring to Figure 20, Human CD45+ cells were not detectable in eight NOD.SCID mice that received the control human Fc protein. In contrast, two of three mice treated with hCD47-Fc protein displayed clear evidence of human HSC engraftment. These data are consistent with the idea that treatment with protein or small molecule agonists of SIRPα can promote engraftment of human HSC in settings of clinical transplantation.

The present invention relates to the following items:
1. An isolated polypeptide selected from the group consisting of:
   a) a polypeptide consisting of the amino acid sequence of SEQ ID NO. 1;
   b) a polypeptide consisting of a fragment of the amino acid sequence of SEQ ID NO. 1, wherein the fragment comprises at least one of residues 31, 32, 34, 37, 74, 77, 83, 84, 86, 87, 90, 91, 96, 100, 102, 114, 118, 126 of SEQ ID NO. 1; and
   c) one of the polypeptide in a) and b) with up to 1 amino acid insertion, deletion or substitution for every 7 amino acids in length of the polypeptide, wherein the polypeptide comprises at least one of residues 31, 32, 34, 37, 74, 77, 83, 84, 86, 87, 90, 91, 96, 100, 102, 114, 118, 126 of SEQ ID NO. 1 and binds human CD47.
2. An isolated polypeptide selected from the group consisting of:
   a) a polypeptide consisting of the amino acid sequence of SEQ ID NO. 2;
   b) a polypeptide consisting of a fragment of the amino acid sequence of SEQ ID NO. 2; and
   c) one of the polypeptide in a) and b) with up to 1 amino acid insertion, deletion or substitution for every 7 amino acids in length of the polypeptide;
      wherein:
      i. at least one of residues at positions 31, 32, 34, 37, 74, 77, 83, 84, 86, 87, 90, 91, 96, 100, 102, 114, 118, 126 of SEQ ID NO. 2 in the polypeptide is replaced with corresponding residues 31, 32, 34, 37, 74, 77, 83, 84, 86, 87, 90, 91, 96, 100, 102, 114, 118, 126 of SEQ ID NO. 1; or
      ii. at least one of residues 129 and 130 of SEQ ID NO. 2 in the polypeptide is deleted.
3. An isolated polypeptide selected from the group consisting of:
   a) a polypeptide consisting of an amino acid sequence selected from the group consisting of SEQ ID NOS. 4-13;
   b) a polypeptide consisting of a fragment of an amino acid sequence selected from the group consisting of SEQ ID NOS. 4-13; and
   c) one of the polypeptide in a) and b) with up to 1 amino acid insertion, deletion or substitution for every 7 amino acids in length of the polypeptide, wherein the polypeptide binds human CD47.
4. The polypeptide of item 1, wherein the polypeptide is the fragment and comprises at least 3 consecutive amino acids in at least one of a region between residues 50-57, 63-71, 74-80, 88-92, 95-100, 103-109, 114-125 or 128-141, inclusive of SEQ ID NO. 1.
5. The polypeptide of item 2, wherein the polypeptide is the fragment and comprises at least 3 consecutive amino acids in at least one of a region between residues 50-57, 63-71, 74-80, 88-92, 95-100, 103-109, 114-125 or 128-143, inclusive of SEQ ID NO. 2.
6. The polypeptide of item 3, wherein the polypeptide is the fragment and comprises at least 3 consecutive amino acids in at least one of a region between residues 24-31, 37-45, 48-54, 62-66, 69-74, 77-83, 88-99 or 102-116, inclusive, of any one of SEQ ID NOs. 4, 7, 8, 9 and 12; or between residues 24-31, 37-45, 48-54, 62-66, 69-74, 77-83, 88-99 or 102-115, inclusive, of SEQ ID NOs. 5, 6, 10, 11 and 13.
7. The polypeptide of any one of items 1 to 6, wherein the amino acid insertion, deletion or substitution is a conservative amino acid substitution.
8. The polypeptide of any one of items 1 to 6, having no amino acid insertion, deletion or substitution.
9. The polypeptide of any one of items 1 to 6, wherein the polypeptide binds human CD47.
10. The polypeptide of any one of items 1 to 9, wherein the polypeptide is the fragment and is between 6 and 30 amino acids in length.
11. The polypeptide of item 10, wherein the fragment is between 8 and 28 amino acids in length.
12. The polypeptide of item 11, wherein the fragment is between 10 and 26 amino acids in length.
13. The polypeptide of item 12, wherein the fragment is between 12 and 24 amino acids in length.
14. The polypeptide of item 13, wherein the fragment is between 14 and 22 amino acids in length.
15. A polypeptide comprising the polypeptide of any one of items 1-14 fused to a second polypeptide.
16. The polypeptide of item 15, wherein the second protein is the Fc portion of IgG.
17. A pharmaceutical composition comprising the polypeptide of any one of items 1-16 and a pharmaceutically acceptable carrier.
18. A method for increasing hematopoietic stem cell engraftment in a mammal comprising administering to the mammal a therapeutically effective amount of the polypeptide of any one of items 1-16.
19. The method of item 18, wherein the increased hematopoietic stem cell engraftment results from suppression of macrophages.
20. Use of the polypeptide of any one of items 1-16, for increasing hematopoietic stem cell engraftment in a mammal.
21. Use of the polypeptide of any one of items 1-16, in the preparation of a medicament for increasing hematopoietic stem cell engraftment in a mammal.
22. The polypeptide of any one of items 1-16, for increasing hematopoietic stem cell engraftment in a mammal.
23. An isolated nucleic acid comprising a sequence that encodes the polypeptide of any one of items 1-16.
24. An expression vector comprising the nucleic acid of item 23 operably linked to an expression control sequence.
25. A cultured cell comprising the vector of item 24.
26. A method of producing a polypeptide comprising culturing the cell of item 25 under conditions permitting expression of the polypeptide.
27. A method for increasing hematopoietic stem cell engraftment in a human comprising modulating the interaction between human Sirpα and human CD47.
28. The method of item 27, wherein the interaction between human Sirpα and human CD47 is modulated by administering a therapeutically effective amount of at least one of:
   a) a polypeptide capable of binding to the extracellular domain of human CD47;
      and
   b) antibodies to human CD47.
29. The method of item 28, wherein the polypeptide capable of binding to the extracellular domain of human CD47 comprises soluble human Sirpα, or a fragment thereof.
30. The method of item 28, wherein the polypeptide is the extracellular domain of human Sirpα.
31. The method of any one of items 29 and 30, wherein the polypeptide is fused to a second protein.
32. The method of item 31, wherein the second protein is the Fc portion of IgG.
33. The method of item 27, wherein the interaction between human Sirpα and human CD47 is modulated by administering a therapeutically effective amount of at least one of:
   a) a polypeptide capable of binding to the extracellular domain of human Sirpα;
      and
   b) antibodies to human Sirpα.
34. The method of item 33, wherein the polypeptide capable of binding to the extracellular domain of Sirpα comprises soluble human CD47, or a fragment thereof.
35. The method of item 34, wherein the polypeptide is the extracellular domain of human Sirpα.
36. The method of any one of items 34 and 35, wherein the polypeptide is fused to a second protein.
37. The method of item 36, wherein the second protein is the Fc portion of IgG.
38. The method of any one of items 27-37, wherein the increased hematopoietic stem cell engraftment results from suppression of macrophages.
39. Use of a compound for increasing hematopoietic stem cell engraftment in a human, the compound comprising:
   a) a polypeptide capable of binding to the extracellular domain of human CD47;
      and
   b) antibodies to human CD47.
40. Use of a compound in the preparation of a medicament for increasing hematopoietic stem cell engraftment in a human, the compound comprising:
   a) a polypeptide capable of binding to the extracellular domain of human CD47;
      and
   b) antibodies to human CD47.
41. The use of any one of items 39 and 40, wherein the polypeptide capable of binding to the extracellular domain of human CD47 comprises soluble human Sirpα, or a fragment thereof.
42. The use of item 41, wherein the polypeptide is the extracellular domain of human Sirpα.
43. The use of any one of items 41 and 42, wherein the polypeptide is fused to a second protein.
44. The use of item 43, wherein the second protein is the Fc portion of IgG.
45. Use of a compound for increasing hematopoietic stem cell engraftment in a human, the compound comprising:
   a) a polypeptide capable of binding to the extracellular domain of human Sirpα;
      and
   b) antibodies to human Sirpα.
46. Use of a compound in the preparation of a medicament for increasing hematopoietic stem cell engraftment in a human, the compound comprising:
   a) a polypeptide capable of binding to the extracellular domain of human Sirpα;
      and
   b) antibodies to human Sirpα.
47. The use of any one of items 45 and 46, wherein the polypeptide capable of binding to the extracellular domain of Sirpα comprises soluble human CD47, or a fragment thereof.
48. The use of item 47, wherein the polypeptide is the extracellular domain of human Sirpα.
49. The use of any one of items 47 and 48, wherein the polypeptide is fused to a second protein.
50. The use of item 49, wherein the second protein is the Fc portion of IgG.
51. The use of any one of items 39-50, additionally for suppression of macrophages.
52. A polypeptide comprising the extracellular domain of CD47 fused to the Fc portion of IgG.
53. The polypeptide of 52, wherein the said polypeptide is encoded consecutively by SEQ ID NOs. 124, 125 and 126.
54. A pharmaceutical composition comprising the polypeptide of any one of items 52 and 53 and a pharmaceutically acceptable carrier.
55. The polypeptide of any one of items 52 and 53 for increasing hematopoietic stem cell engraftment in a human.
56. An isolated nucleic acid comprising a sequence that encodes the polypeptide of any one of items 52 and 53.
57. An expression vector comprising the nucleic acid of item 56 operably linked to an expression control sequence.
58. The expression vector of item 57, further comprising a Kozak consensus sequence of nucleic acids.
59. A cultured cell comprising the vector of any on of items 57 and 58.
60. A method of producing a polypeptide comprising culturing the cell of item 59 under conditions permitting expression of the polypeptide.
61. A method of identifying a compound that increases hematopoietic stem cell engraftment in a human comprising:
   a) contacting at least one of the extracellular domain of human Sirpα and human CD47 with at least one test compound;
   b) determining the at least one test compound as binding to the at least one of human Sirpα and human CD47;
   c) contacting the test compound with human hematopoietic cells in a stromal environment; and
   d) determining whether hematopoietic stem cell engraftment is increased in the presence of the test compound.
62. A method of determining genetic polymorphisms in humans affecting hematopoietic stem cell engraftment comprising:
   a) sequencing the Sirpα gene from a plurality of humans having undergone hematopoietic transplantation;
   b) determining nucleotide differences in the Sirpα gene within the plurality of humans; and
   c) correlating the nucleotide differences with hematopoietic stem cell engraftment to determine relevant polymorphisms.
63. The method of item 62, wherein the nucleotide differences result in amino acid differences.
64. A method of determining likelihood of hematopoietic stem cell engraftment in a recipient comprising:
   a) sequencing the Sirpα gene from the recipient; and
   b) determining whether the relevant polymorphisms of item 62 exist.
65. The method of item 64, wherein the nucleotide differences result in amino acid differences.
66. The method of item 65, wherein the amino acid differences is at least one of:
   a) replacement of at least one of residues at positions 31, 32, 34, 37, 74, 77, 83, 84, 86, 87, 90, 91, 96, 100, 102, 114, 118, 126 of SEQ ID NO. 2 with corresponding residues 31, 32, 34, 37, 74, 77, 83, 84, 86, 87, 90, 91, 96, 100, 102, 114, 118, 126 of SEQ ID NO. 1; or
   b) deletion of at least one of residues 129 and 130 of SEQ ID NO. 2.
   Although preferred embodiments of the invention have been described herein, it will be understood by those skilled in the art that variations may be made thereto without departing from the spirit of the invention or the scope of the appended claims. All documents disclosed herein are incorporated by reference.

## Claims

1. Compound for use in increasing hematopoietic stem cell engraftment in a human, the compound being a polypeptide capable of binding to the extracellular domain of human Sirpα or an antibody to human Sirpα.

2. The compound of claim 1, wherein the polypeptide capable of binding to the extracellular domain of Sirpα comprises soluble human CD47, or a fragment thereof.

3. The compound of claim 2, wherein the polypeptide is the extracellular domain of human CD47,

4. The compound of claim 3, wherein the polypeptide is the extracellular domain of human CD47 fused to a second protein.

5. The compound of claim 4, wherein the polypeptide is the extracellular domain of human CD47 fused to the Fc portion of IgG.

6. The compound of any one of claims 1-5, additionally for suppression of macrophages.

7. A polypeptide comprising the extracellular domain of CD47 fused to the Fc portion of IgG.

8. The polypeptide of 7, wherein the said polypeptide is encoded consecutively by SEQ ID NOs. 124, 125 and 126.

9. A pharmaceutical composition comprising the polypeptide of any one of claims 7 and 8 and a pharmaceutically acceptable carrier.

10. The polypeptide of any one of claims 7 and 8 for use in increasing hematopoietic stem cell engraftment in a human.


SEQUENCE LISTING

<110> University Health Network

<120> Modulation of SIRPalpha - CD47 Interaction For Increasing Human
Hematopoetic Stem Cell Engraftment and Compounds Therefor

<130> 05014971-26EP

<140> PCT/CA2008/001814
<141> 2008-10-10

<150> US 60/960,724
<151> 2007-10-11

<160> 126

<170> PatentIn version 3.5

<210> 1
<211> 507
<212> PRT
<213> Mouse

<400> 1

Met Glu Pro Ala Gly Pro Ala Pro Gly Arg Leu Gly Pro Leu Leu Leu
1               5                   10                  15

Cys Leu Leu Leu Ser Ala Ser Cys Phe Cys Thr Gly Ala Thr Arg Thr
            20                  25                  30

Glu Val Lys Val Ile Gln Pro Glu Lys Ser Val Ser Val Ala Ala Gly
        35                  40                  45

Asp Ser Thr Val Leu Asn Cys Thr Leu Thr Ser Leu Leu Pro Val Gly
    50                  55                  60

Pro Ile Arg Trp Tyr Arg Gly Val Gly Gln Ser Arg Gln Leu Ile Tyr
65                  70                  75                  80

Ser Phe Thr Thr Glu His Phe Pro Arg Val Thr Asn Val Ser Asp Ala
                85                  90                  95

Thr Lys Arg Ser Asn Leu Asp Phe Ser Ile Arg Ile Ser Asn Val Thr
            100                 105                 110

Pro Glu Asp Ala Gly Thr Tyr Tyr Cys Val Lys Phe Gln Arg Gly Ser
        115                 120                 125

Pro Asp Thr Glu Ile Gln Ser Gly Gly Gly Thr Glu Val Tyr Val Leu
    130                 135                 140

Ala Lys Pro Ser Pro Pro Glu Val Ser Gly Pro Ala Asp Arg Gly Ile
145                 150                 155                 160

Pro Asp Gln Lys Val Asn Phe Thr Cys Lys Ser His Gly Phe Ser Pro
                165                 170                 175

Arg Asn Ile Thr Leu Lys Trp Phe Lys Asp Gly Gln Glu Leu His Pro
            180                 185                 190

Leu Glu Thr Thr Val Asn Pro Ser Gly Lys Asn Val Ser Tyr Asn Ile
        195                 200                 205

Ser Ser Thr Val Arg Val Val Leu Asn Ser Met Asp Val His Ser Lys
    210                 215                 220

Val Ile Cys Glu Val Ala His Ile Thr Leu Asp Arg Ser Pro Leu Arg
225                 230                 235                 240

Gly Ile Ala Asn Leu Ser Asn Phe Ile Arg Val Ser Pro Thr Val Lys
                245                 250                 255

Val Thr Gln Gln Ser Pro Thr Ser Met Ser Gln Val Asn Leu Thr Cys
            260                 265                 270

Arg Ala Glu Arg Phe Tyr Pro Glu Asp Leu Gln Leu Ile Trp Leu Glu
        275                 280                 285

Asn Gly Asn Val Ser Arg Asn Asp Thr Pro Lys Asn Leu Thr Lys Asn
    290                 295                 300

Thr Asp Gly Thr Tyr Asn Tyr Thr Ser Leu Phe Leu Val Asn Ser Ser
305                 310                 315                 320

Ala His Arg Glu Asp Val Val Phe Thr Cys Gln Val Lys His Asp Gln
                325                 330                 335

Gln Pro Ala Ile Thr Arg Asn His Thr Val Leu Gly Leu Ala His Ser
            340                 345                 350

Ser Asp Gln Gly Ser Met Gln Thr Phe Pro Gly Asn Asn Ala Thr His
        355                 360                 365

Asn Trp Asn Val Phe Ile Gly Val Gly Val Ala Cys Ala Leu Leu Val
    370                 375                 380

Val Leu Leu Met Ala Ala Leu Tyr Leu Leu Arg Ile Lys Gln Lys Lys
385                 390                 395                 400

Ala Lys Gly Ser Thr Ser Ser Thr Arg Leu His Glu Pro Glu Lys Asn
                405                 410                 415

Ala Arg Glu Ile Thr Gln Ile Gln Asp Thr Asn Asp Ile Asn Asp Ile
            420                 425                 430

Thr Tyr Ala Asp Leu Asn Leu Pro Lys Glu Lys Lys Pro Ala Pro Arg
        435                 440                 445

Ala Pro Glu Pro Asn Asn His Thr Glu Tyr Ala Ser Ile Glu Thr Gly
    450                 455                 460

Lys Val Pro Arg Pro Glu Asp Thr Leu Thr Tyr Ala Asp Leu Asp Met
465                 470                 475                 480

Val His Leu Ser Arg Ala Gln Pro Ala Pro Lys Pro Glu Pro Ser Phe
                485                 490                 495

Ser Glu Tyr Ala Ser Val Gln Val Gln Arg Lys
            500                 505

<210>  2
<211>  509
<212>  PRT
<213>  Mouse

<400>  2

Met Glu Pro Ala Gly Pro Ala Pro Gly Arg Leu Gly Pro Leu Leu Leu
1               5                   10                  15

Cys Leu Leu Leu Ser Ala Ser Cys Phe Cys Thr Gly Ala Thr Gly Lys
            20                  25                  30

Glu Leu Lys Val Thr Gln Pro Glu Lys Ser Val Ser Val Ala Ala Gly
        35                  40                  45

Asp Ser Thr Val Leu Asn Cys Thr Leu Thr Ser Leu Leu Pro Val Gly
    50                  55                  60

Pro Ile Arg Trp Tyr Arg Gly Val Gly Pro Ser Arg Leu Leu Ile Tyr
65                  70                  75                  80

Ser Phe Ala Gly Glu Tyr Val Pro Arg Ile Arg Asn Val Ser Asp Thr
                85                  90                  95

Thr Lys Arg Asn Asn Met Asp Phe Ser Ile Arg Ile Ser Asn Val Thr
            100                 105                 110

Pro Ala Asp Ala Gly Ile Tyr Tyr Cys Val Lys Phe Gln Lys Gly Ser
        115                 120                 125

Ser Glu Pro Asp Thr Glu Ile Gln Ser Gly Gly Gly Thr Glu Val Tyr
    130                 135                 140

Val Leu Ala Lys Pro Ser Pro Pro Glu Val Ser Gly Pro Ala Asp Arg
145                 150                 155                 160

Gly Ile Pro Asp Gln Lys Val Asn Phe Thr Cys Lys Ser His Gly Phe
                165                 170                 175

Ser Pro Arg Asn Ile Thr Leu Lys Trp Phe Lys Asp Gly Gln Glu Leu
            180                 185                 190

His Pro Leu Glu Thr Thr Val Asn Pro Ser Gly Lys Asn Val Ser Tyr
        195                 200                 205

Asn Ile Ser Ser Thr Val Arg Val Val Leu Asn Ser Met Asp Val Asn
    210                 215                 220

Ser Lys Val Ile Cys Glu Val Ala His Ile Thr Leu Asp Arg Ser Pro
225                 230                 235                 240

Leu Arg Gly Ile Ala Asn Leu Ser Asn Phe Ile Arg Val Ser Pro Thr
                245                 250                 255

Val Lys Val Thr Gln Gln Ser Pro Thr Ser Met Asn Gln Val Asn Leu
            260                 265                 270

Thr Cys Arg Ala Glu Arg Phe Tyr Pro Glu Asp Leu Gln Leu Ile Trp
        275                 280                 285

Leu Glu Asn Gly Asn Val Ser Arg Asn Asp Thr Pro Lys Asn Leu Thr
    290                 295                 300

Lys Asn Thr Asp Gly Thr Tyr Asn Tyr Thr Ser Leu Phe Leu Val Asn
305                 310                 315                 320

Ser Ser Ala His Arg Glu Asp Val Val Phe Thr Cys Gln Val Lys His
                325                 330                 335

Asp Gln Gln Pro Ala Ile Thr Arg Asn His Thr Val Leu Gly Phe Ala
            340                 345                 350

His Ser Ser Asp Gln Gly Ser Met Gln Thr Phe Pro Asp Asn Asn Ala
        355                 360                 365

Thr His Asn Trp Asn Val Phe Ile Gly Val Gly Val Ala Cys Ala Leu
    370                 375                 380

Leu Val Val Leu Leu Met Ala Ala Leu Tyr Leu Leu Arg Ile Lys Gln
385                 390                 395                 400

Lys Lys Ala Lys Gly Ser Thr Ser Ser Thr Arg Leu His Glu Pro Glu
                405                 410                 415

Lys Asn Ala Arg Glu Ile Thr Gln Ile Gln Asp Thr Asn Asp Ile Asn
            420                 425                 430

Asp Ile Thr Tyr Ala Asp Leu Asn Leu Pro Lys Glu Lys Lys Pro Ala
        435                 440                 445

Pro Arg Ala Pro Glu Pro Asn Asn His Thr Glu Tyr Ala Ser Ile Glu
    450                 455                 460

Thr Gly Lys Val Pro Arg Pro Glu Asp Thr Leu Thr Tyr Ala Asp Leu
465                 470                 475                 480

Asp Met Val His Leu Ser Arg Ala Gln Pro Ala Pro Lys Pro Glu Pro
                485                 490                 495

Ser Phe Ser Glu Tyr Ala Ser Val Gln Val Gln Arg Lys
            500                 505

<210>  3
<211>  513
<212>  PRT
<213>  Mouse

<400>  3

Met Glu Pro Ala Gly Pro Ala Pro Gly Arg Leu Gly Pro Leu Leu Leu
1               5                   10                  15

Cys Leu Leu Leu Ser Ala Ser Cys Phe Cys Thr Gly Ala Thr Gly Lys
            20                  25                  30

Glu Leu Lys Val Thr Gln Pro Glu Lys Ser Val Ser Val Ala Ala Gly
        35                  40                  45

Asp Ser Thr Val Leu Asn Cys Thr Leu Thr Ser Leu Leu Pro Val Gly
    50                  55                  60

Pro Ile Arg Trp Tyr Arg Gly Val Gly Pro Ser Arg Leu Leu Ile Tyr
65                  70                  75                  80

Ser Phe Ala Gly Glu Tyr Val Pro Arg Ile Arg Asn Val Ser Asp Thr
                85                  90                  95

Thr Lys Arg Asn Asn Met Asp Phe Ser Ile Arg Ile Ser Asn Val Thr
            100                 105                 110

Pro Ala Asp Ala Gly Ile Tyr Tyr Cys Val Lys Phe Gln Lys Gly Ser
        115                 120                 125

Ser Glu Pro Asp Thr Glu Ile Gln Ser Gly Gly Gly Thr Glu Val Tyr
    130                 135                 140

Val Leu Ala Lys Pro Ser Pro Pro Glu Val Ser Gly Pro Ala Asp Arg
145                 150                 155                 160

Gly Ile Pro Asp Gln Lys Val Asn Phe Thr Cys Lys Ser His Gly Phe
                165                 170                 175

Ser Pro Arg Asn Ile Thr Leu Lys Trp Phe Lys Asp Gly Gln Glu Leu
            180                 185                 190

His Pro Leu Glu Thr Thr Val Asn Pro Ser Gly Lys Asn Val Ser Tyr
        195                 200                 205

Asn Ile Ser Ser Thr Val Arg Val Val Leu Asn Ser Met Asp Val Asn
    210                 215                 220

Ser Lys Val Ile Cys Glu Val Ala His Ile Thr Leu Asp Arg Ser Pro
225                 230                 235                 240

Leu Arg Gly Ile Ala Asn Leu Ser Asn Phe Ile Arg Val Ser Pro Thr
                245                 250                 255

Val Lys Val Thr Gln Gln Ser Pro Thr Ser Met Asn Gln Val Asn Leu
            260                 265                 270

Thr Cys Arg Ala Glu Arg Phe Tyr Pro Glu Asp Leu Gln Leu Ile Trp
        275                 280                 285

Leu Glu Asn Gly Asn Val Ser Arg Asn Asp Thr Pro Lys Asn Leu Thr
    290                 295                 300

Lys Asn Thr Asp Gly Thr Tyr Asn Tyr Thr Ser Leu Phe Leu Val Asn
305                 310                 315                 320

Ser Ser Ala His Arg Glu Asp Val Val Phe Thr Cys Gln Val Lys His
                325                 330                 335

Asp Gln Gln Pro Ala Ile Thr Arg Asn His Thr Val Leu Gly Phe Ala
            340                 345                 350

His Ser Ser Asp Gln Gly Ser Met Gln Thr Phe Pro Asp Asn Asn Ala
        355                 360                 365

Thr His Asn Trp Asn Val Phe Ile Gly Val Gly Val Ala Cys Ala Leu
    370                 375                 380

Leu Val Val Leu Leu Met Ala Ala Leu Tyr Leu Leu Arg Ile Lys Gln
385                 390                 395                 400

Lys Lys Ala Lys Gly Ser Thr Ser Ser Thr Arg Leu His Glu Pro Glu
                405                 410                 415

Lys Asn Ala Arg Glu Ile Thr Gln Val Gln Ser Leu Ile Gln Asp Thr
            420                 425                 430

Asn Asp Ile Asn Asp Ile Thr Tyr Ala Asp Leu Asn Leu Pro Lys Glu
        435                 440                 445

Lys Lys Pro Ala Pro Arg Ala Pro Glu Pro Asn Asn His Thr Glu Tyr
    450                 455                 460

Ala Ser Ile Glu Thr Gly Lys Val Pro Arg Pro Glu Asp Thr Leu Thr
465                 470                 475                 480

Tyr Ala Asp Leu Asp Met Val His Leu Ser Arg Ala Gln Pro Ala Pro
                485                 490                 495

Lys Pro Glu Pro Ser Phe Ser Glu Tyr Ala Ser Val Gln Val Gln Arg
            500                 505                 510

Lys

<210>  4
<211>  120
<212>  PRT
<213>  Human

<400>  4

Gly Val Ala Gly Glu Glu Glu Leu Gln Val Ile Gln Pro Asp Lys Ser
1               5                   10                  15

Val Leu Val Ala Ala Gly Glu Thr Ala Thr Leu Arg Cys Thr Ala Thr
            20                  25                  30

Ser Leu Ile Pro Val Gly Pro Ile Gln Trp Phe Arg Gly Ala Gly Pro
        35                  40                  45

Gly Arg Glu Leu Ile Tyr Asn Gln Lys Glu Gly His Phe Pro Arg Val
    50                  55                  60

Thr Thr Val Ser Asp Leu Thr Lys Arg Asn Asn Met Asp Phe Ser Ile
65                  70                  75                  80

Arg Ile Gly Asn Ile Thr Pro Ala Asp Ala Gly Thr Tyr Tyr Cys Val
                85                  90                  95

Lys Phe Arg Lys Gly Ser Pro Asp Asp Val Glu Phe Lys Ser Gly Ala
            100                 105                 110

Gly Thr Glu Leu Ser Val Arg Ala
        115                 120

<210>  5
<211>  119
<212>  PRT
<213>  Human

<400>  5

Gly Val Ala Gly Glu Glu Glu Leu Gln Val Ile Gln Pro Asp Lys Ser
1               5                   10                  15

Val Ser Val Ala Ala Gly Glu Ser Ala Ile Leu His Cys Thr Val Thr
            20                  25                  30

Ser Leu Ile Pro Val Gly Pro Ile Gln Trp Phe Arg Gly Ala Gly Pro
        35                  40                  45

Ala Arg Glu Leu Ile Tyr Asn Gln Lys Glu Gly His Phe Pro Arg Val
    50                  55                  60

Thr Thr Val Ser Glu Ser Thr Lys Arg Glu Asn Met Asp Phe Ser Ile
65                  70                  75                  80

Ser Ile Ser Asn Ile Thr Pro Ala Asp Ala Gly Thr Tyr Tyr Cys Val
                85                  90                  95

Lys Phe Arg Lys Gly Ser Pro Asp Thr Glu Phe Lys Ser Gly Ala Gly
            100                 105                 110

Thr Glu Leu Ser Val Arg Ala
        115

<210> 6
<211> 119
<212> PRT
<213> Human

<400> 6

Gly Val Ala Gly Glu Glu Glu Leu Gln Val Ile Gln Pro Asp Lys Ser
1               5                   10                  15

Val Ser Val Ala Ala Gly Glu Ser Ala Ile Leu Leu Cys Thr Val Thr
            20                  25                  30

Ser Leu Ile Pro Val Gly Pro Ile Gln Trp Phe Arg Gly Ala Gly Pro
        35                  40                  45

Ala Arg Glu Leu Ile Tyr Asn Gln Lys Glu Gly His Phe Pro Arg Val
    50                  55                  60

Thr Thr Val Ser Glu Ser Thr Lys Arg Glu Asn Met Asp Phe Ser Ile
65                  70                  75                  80

Ser Ile Ser Asn Ile Thr Pro Ala Asp Ala Gly Thr Tyr Tyr Cys Val
                85                  90                  95

Lys Phe Arg Lys Gly Ser Pro Asp Thr Glu Phe Lys Ser Gly Ala Gly
            100                 105                 110

Thr Glu Leu Ser Val Arg Ala
        115

<210>  7
<211>  120
<212>  PRT
<213>  Human

<400>  7

Gly Val Ala Gly Glu Glu Gly Leu Gln Val Ile Gln Pro Asp Lys Ser
1               5                   10                  15

Val Ser Val Ala Ala Gly Glu Ser Ala Ile Leu His Cys Thr Ala Thr
            20                  25                  30

Ser Leu Ile Pro Val Gly Pro Ile Gln Trp Phe Arg Gly Ala Gly Pro
        35                  40                  45

Gly Arg Glu Leu Ile Tyr Asn Gln Lys Glu Gly His Phe Pro Arg Val
    50                  55                  60

Thr Thr Val Ser Asp Leu Thr Lys Arg Asn Asn Met Asp Phe Ser Ile
65                  70                  75                  80

Arg Ile Gly Asn Ile Thr Pro Ala Asp Ala Gly Thr Tyr Tyr Cys Val
                85                  90                  95

Lys Phe Arg Lys Gly Ser Pro Asp Asp Val Glu Phe Lys Ser Gly Ala
            100                 105                 110

Gly Thr Glu Leu Ser Val Arg Ala
        115                 120


<210>  8
<211>  120
<212>  PRT
<213>  Human

<400>  8

Gly Val Ala Gly Glu Glu Glu Leu Gln Val Ile Gln Pro Asp Lys Phe
1               5                   10                  15

Val Leu Val Ala Ala Gly Glu Thr Ala Thr Leu Arg Cys Thr Ala Thr
            20                  25                  30

Ser Leu Ile Pro Val Gly Pro Ile Gln Trp Phe Arg Gly Ala Gly Pro
        35                  40                  45

Gly Arg Glu Leu Ile Tyr Asn Gln Lys Glu Gly His Phe Pro Arg Val
    50                  55                  60

Thr Thr Val Ser Asp Leu Thr Lys Arg Asn Asn Met Asp Phe Ser Ile
65                  70                  75                  80

Arg Ile Gly Asn Ile Thr Pro Ala Asp Ala Gly Thr Tyr Tyr Cys Val

85                  90                  95

Lys Phe Arg Lys Gly Ser Pro Asp Asp Val Glu Phe Lys Ser Gly Ala
            100                 105                 110

Gly Thr Glu Leu Ser Val Arg Ala
        115                 120

<210>  9
<211>  120
<212>  PRT
<213>  Human

<400>  9

Gly Val Ala Gly Glu Glu Glu Leu Gln Val Ile Gln Pro Asp Lys Ser
1               5                   10                  15

Val Leu Val Ala Ala Gly Glu Thr Ala Thr Leu Arg Cys Thr Ala Thr
            20                  25                  30

Ser Leu Ile Pro Val Gly Pro Ile Gln Trp Phe Arg Gly Ala Gly Pro
        35                  40                  45

Gly Arg Glu Leu Ile Tyr Asn Gln Lys Glu Gly His Phe Pro Arg Val
    50                  55                  60

Thr Thr Val Ser Asp Leu Thr Lys Arg Asn Asn Met Asp Phe Pro Ile
65                  70                  75                  80

Arg Ile Gly Asn Ile Thr Pro Ala Asp Ala Gly Thr Tyr Tyr Cys Val
                85                  90                  95

Lys Phe Arg Lys Gly Ser Pro Asp Asp Val Glu Phe Lys Ser Gly Ala
            100                 105                 110

Gly Thr Glu Leu Ser Val Arg Ala
        115                 120

<210>  10
<211>  120
<212>  PRT
<213>  Human

<400>  10

Gly Val Ala Gly Glu Glu Glu Leu Gln Val Ile Gln Pro Asp Lys Ser
1               5                   10                  15

Val Ser Val Ala Ala Gly Glu Ser Ala Ile Leu His Cys Thr Val Thr
            20                  25                  30

Ser Leu Ile Pro Val Gly Pro Ile Gln Trp Phe Arg Gly Ala Gly Pro
        35                  40                  45

Ala Arg Glu Leu Ile Tyr Asn Gln Lys Glu Gly His Phe Pro Arg Val
    50                  55                  60

Thr Thr Val Ser Glu Ser Thr Lys Arg Glu Asn Met Asp Phe Ser Ile
65                  70                  75                  80

Ser Ile Ser Asn Ile Thr Pro Ala Asp Ala Gly Thr Tyr Tyr Cys Val
                85                  90                  95

Lys Phe Arg Lys Gly Ser Pro Asp Asp Val Glu Phe Lys Ser Gly Ala
            100                 105                 110

Gly Thr Glu Leu Ser Val Arg Ala
        115                 120

<210>  11
<211>  119
<212>  PRT
<213>  Human

<400>  11

Gly Val Ala Gly Glu Glu Glu Leu Gln Val Ile Gln Pro Asp Lys Ser
1               5                   10                  15

Val Leu Val Ala Ala Gly Glu Thr Ala Thr Leu Arg Cys Thr Ala Thr
            20                  25                  30

Ser Leu Ile Pro Val Gly Pro Ile Gln Trp Phe Arg Gly Ala Gly Pro
        35                  40                  45

Ala Arg Glu Leu Ile Tyr Asn Gln Lys Glu Gly His Phe Pro Arg Val
    50                  55                  60

Thr Thr Val Ser Glu Ser Thr Lys Arg Glu Asn Met Asp Phe Ser Ile
65                  70                  75                  80

Ser Ile Ser Asn Ile Thr Pro Ala Asp Ala Gly Thr Tyr Tyr Cys Val
                85                  90                  95

Lys Phe Arg Lys Gly Ser Pro Asp Thr Glu Phe Lys Ser Gly Ala Gly
            100                 105                 110

Thr Glu Leu Ser Val Arg Ala
        115

<210>  12
<211>  120
<212>  PRT
<213>  Human

<400>  12

Gly Val Ala Gly Glu Glu Glu Leu Gln Val Ile Gln Pro Asp Lys Ser

1                   5                   10                  15

Val Leu Val Ala Ala Gly Glu Thr Ala Thr Leu Arg Cys Thr Ala Thr
                20                  25                  30

Ser Leu Ile Pro Val Gly Pro Ile Gln Trp Phe Arg Gly Ala Gly Pro
        35                  40                  45

Gly Arg Glu Leu Ile Tyr Asn Gln Lys Glu Gly His Phe Pro Arg Val
    50                  55                  60

Thr Thr Val Ser Asp Leu Thr Lys Arg Asn Asn Met Asp Phe Ser Ile
65                  70                  75                  80

Arg Ile Ser Asn Ile Thr Pro Ala Asp Ala Gly Thr Tyr Tyr Cys Val
                85                  90                  95

Lys Phe Arg Lys Gly Ser Pro Asp Asp Val Glu Phe Lys Ser Gly Ala
            100                 105                 110

Gly Thr Glu Leu Ser Val Arg Ala
        115                 120

<210>  13
<211>  119
<212>  PRT
<213>  Human

<400>  13

Arg Val Ala Gly Glu Glu Glu Leu Gln Val Ile Gln Pro Asp Lys Ser
1                   5                   10                  15

Val Ser Val Ala Ala Gly Glu Ser Ala Ile Leu His Cys Thr Val Thr
                20                  25                  30

Ser Leu Ile Pro Val Gly Pro Ile Gln Trp Phe Arg Gly Ala Gly Pro
        35                  40                  45

Ala Arg Glu Leu Ile Tyr Asn Gln Lys Glu Gly His Phe Pro Arg Val
    50                  55                  60

Thr Thr Val Ser Glu Ser Thr Lys Arg Glu Asn Met Asp Phe Ser Ile
65                  70                  75                  80

Ser Ile Ser Asn Ile Thr Pro Ala Asp Ala Gly Thr Tyr Tyr Cys Val
                85                  90                  95

Lys Phe Arg Lys Gly Ser Pro Asp Thr Glu Phe Lys Ser Gly Ala Gly
            100                 105                 110

Thr Glu Leu Ser Val Arg Ala
        115

<210>  14
<211>  119
<212>  PRT
<213>  Mouse

<400>  14

Ala Thr Gly Lys Glu Leu Lys Val Thr Gln Pro Glu Lys Ser Val Ser
1               5                   10                  15

Val Ala Ala Gly Asp Ser Thr Val Leu Asn Cys Thr Leu Thr Ser Leu
            20                  25                  30

Leu Pro Val Gly Pro Ile Arg Trp Tyr Arg Gly Val Gly Pro Ser Arg
        35                  40                  45

Leu Leu Ile Tyr Ser Phe Ala Gly Glu Tyr Val Pro Arg Ile Arg Asn
    50                  55                  60

Val Ser Asp Thr Thr Lys Arg Asn Asn Met Asp Phe Ser Ile Arg Ile
65                  70                  75                  80

Ser Asn Val Thr Pro Ala Asp Ala Gly Ile Tyr Tyr Cys Val Lys Phe
                85                  90                  95

Gln Lys Gly Ser Ser Glu Pro Asp Thr Glu Ile Gln Ser Gly Gly Gly
            100                 105                 110

Thr Glu Val Tyr Val Leu Ala
        115

<210>  15
<211>  119
<212>  PRT
<213>  Mouse

<400>  15

Ala Thr Gly Lys Glu Leu Lys Val Thr Gln Pro Glu Lys Ser Val Ser
1               5                   10                  15

Val Ala Ala Gly Asp Ser Thr Val Leu Asn Cys Thr Leu Thr Ser Leu
            20                  25                  30

Leu Pro Val Gly Pro Ile Arg Trp Tyr Arg Gly Val Gly Pro Ser Arg
        35                  40                  45

Leu Leu Ile Tyr Ser Phe Ala Gly Glu Tyr Val Pro Arg Ile Arg Asn
    50                  55                  60

Val Ser Asp Thr Thr Lys Arg Asn Asn Met Asp Phe Ser Ile Arg Ile
65                  70                  75                  80

Ser Asn Val Thr Pro Ala Asp Ala Gly Ile Tyr Tyr Cys Val Lys Phe
                85                  90                  95

Gln Lys Gly Ser Ser Glu Pro Asp Thr Glu Ile Gln Ser Gly Gly Gly
            100                 105                 110

Thr Glu Val Tyr Val Leu Ala
        115

<210>  16
<211>  117
<212>  PRT
<213>  Mouse

<400>  16

Ala Thr Arg Thr Glu Val Lys Val Ile Gln Pro Glu Lys Ser Val Ser
1               5                   10                  15

Val Ala Ala Gly Asp Ser Thr Val Leu Asn Cys Thr Leu Thr Ser Leu
            20                  25                  30

Leu Pro Val Gly Pro Ile Arg Trp Tyr Arg Gly Val Gly Gln Ser Arg
        35                  40                  45

Gln Leu Ile Tyr Ser Phe Thr Thr Glu His Phe Pro Arg Val Thr Asn
    50                  55                  60

Val Ser Asp Ala Thr Lys Arg Ser Asn Leu Asp Phe Ser Ile Arg Ile
65                  70                  75                  80

Ser Asn Val Thr Pro Glu Asp Ala Gly Thr Tyr Tyr Cys Val Lys Phe
                85                  90                  95

Gln Arg Gly Ser Pro Asp Thr Glu Ile Gln Ser Gly Gly Gly Thr Glu
            100                 105                 110

Val Tyr Val Leu Ala
        115

<210>  17
<211>  119
<212>  PRT
<213>  Mouse

<400>  17

Ala Thr Gly Thr Glu Val Lys Val Thr Gln Pro Glu Lys Ser Val Ser
1               5                   10                  15

Val Ala Ala Gly Asp Ser Thr Ile Leu Asn Cys Thr Val Thr Ser Leu
            20                  25                  30

Leu Pro Val Gly Pro Ile Arg Trp Tyr Arg Gly Val Gly Gln Ser Arg
        35                  40                  45

Leu Leu Ile Tyr Ser Phe Thr Gly Glu His Phe Pro Arg Val Arg Asn
    50                  55                  60

Val Ser Asp Thr Thr Lys Arg Asn Asn Met Asp Phe Ser Ile Arg Ile
65                  70                  75                  80

Ser Asn Val Thr Pro Glu Asp Ala Gly Thr Tyr Tyr Cys Val Lys Phe
                85                  90                  95

Gln Arg Gly Ser Ser Glu Pro Asp Thr Glu Ile Gln Ser Gly Gly Gly
            100                 105                 110

Thr Glu Val Tyr Val Leu Ala
        115

<210>  18
<211>  119
<212>  PRT
<213>  Mouse

<400>  18

Val Thr Gly Lys Glu Leu Lys Val Thr Gln Pro Glu Lys Ser Val Ser
1               5                   10                  15

Val Ala Ala Gly Asp Ser Thr Val Leu Asn Cys Thr Leu Thr Ser Leu
            20                  25                  30

Leu Pro Val Gly Pro Ile Lys Trp Tyr Arg Gly Val Gly Gln Ser Arg
        35                  40                  45

Leu Leu Ile Tyr Ser Phe Thr Gly Glu His Phe Pro Arg Val Thr Asn
    50                  55                  60

Val Ser Asp Ala Thr Lys Arg Asn Asn Met Asp Phe Ser Ile Arg Ile
65                  70                  75                  80

Ser Asn Val Thr Pro Glu Asp Ala Gly Thr Tyr Tyr Cys Val Lys Phe
                85                  90                  95

Gln Lys Gly Pro Ser Glu Pro Asp Thr Glu Ile Gln Ser Gly Gly Gly
            100                 105                 110

Thr Glu Val Tyr Val Leu Ala
        115

<210>  19
<211>  23
<212>  DNA
<213>  Mouse

<400>  19
gaacataggt gcacactcac ata                                              23

<210>  20
<211>  19
<212>  DNA
<213>  Mouse

<400>  20
tgatgctgga ggttgtcct                                                    19


<210>  21
<211>  29
<212>  DNA
<213>  Mouse

<400>  21
gatgaaagag ataagacaga tgttagata                                         29


<210>  22
<211>  26
<212>  DNA
<213>  Mouse

<400>  22
tggttattaa agagctgttt tacatg                                            26


<210>  23
<211>  21
<212>  DNA
<213>  Mouse

<400>  23
aagttggaac tctgcaggac a                                                 21


<210>  24
<211>  20
<212>  DNA
<213>  Mouse

<400>  24
gtgtcttcaa tgcagcacgt                                                   20


<210>  25
<211>  23
<212>  DNA
<213>  Mouse

<400>  25
tattgtttat ggaaattgga ccc                                               23


<210>  26
<211>  22
<212>  DNA
<213>  Mouse

<400>  26
catctctgaa ggaaaaagtg ca                                                22


<210>  27
<211>  25
<212>  DNA

<213>  Mouse

<400>  27
gggagacaac aaataatcat attgc                                          25


<210>  28
<211>  20
<212>  DNA
<213>  Mouse

<400>  28
agaggtgggt cctggaaact                                                20


<210>  29
<211>  20
<212>  DNA
<213>  Mouse

<400>  29
ctgaaaatcg tcccttgacc                                                20


<210>  30
<211>  20
<212>  DNA
<213>  Mouse

<400>  30
caggagcatg aaatggggat                                                20


<210>  31
<211>  20
<212>  DNA
<213>  Mouse

<400>  31
tccacacaag gtgtcctctg                                                20


<210>  32
<211>  20
<212>  DNA
<213>  Mouse

<400>  32
gctcaggtga cctccaaaac                                                20


<210>  33
<211>  24
<212>  DNA
<213>  Mouse

<400>  33
atcaccactg tgggcattct gaac                                           24


<210>  34
<211>  26
<212>  DNA
<213>  Mouse

<400>  34
cagtagaggc caagggcatc agaatc                                         26

<210>  35
<211>  20
<212>  DNA
<213>  Mouse

<400>  35
tgtctcccca aagtagcagg                                                    20


<210>  36
<211>  20
<212>  DNA
<213>  Mouse

<400>  36
gtgatgcagg agtttctgca                                                    20


<210>  37
<211>  20
<212>  DNA
<213>  Mouse

<400>  37
ggcagattta tcgctgtgag                                                    20


<210>  38
<211>  15
<212>  DNA
<213>  Mouse

<400>  38
gtgcgccacc aatgc                                                         15


<210>  39
<211>  21
<212>  DNA
<213>  Mouse

<400>  39
cttcagtgct gtatccactg c                                                  21


<210>  40
<211>  21
<212>  DNA
<213>  Mouse

<400>  40
ctcccactcc cattaattct c                                                  21


<210>  41
<211>  19
<212>  DNA
<213>  Mouse

<400>  41
aggcaattac aaggcctgg                                                     19


<210>  42
<211>  20
<212>  DNA
<213>  Mouse

<400>  42
cacccatctc cctcagtcat                                                    20


<210>  43
<211>  22
<212>  DNA
<213>  Mouse

<400>  43
gtagagactt ggtgggtttt gg                                                 22


<210>  44
<211>  23
<212>  DNA
<213>  Mouse

<400>  44
atctctgctg acaaagaaca gtg                                                23


<210>  45
<211>  20
<212>  DNA
<213>  Mouse

<400>  45
catgtgccat ggtacaaagc                                                    20


<210>  46
<211>  21
<212>  DNA
<213>  Mouse

<400>  46
tggcttgttt cagatagcac a                                                  21


<210>  47
<211>  20
<212>  DNA
<213>  Mouse

<400>  47
tcaccagcct gaaaacactg                                                    20


<210>  48
<211>  23
<212>  DNA
<213>  Mouse

<400>  48
tctgggtaca atccttagtc ctg                                                23


<210>  49
<211>  21
<212>  DNA
<213>  Mouse

<400>  49
gccagataca attttccctc a                                                  21

<210>  50
<211>  18
<212>  DNA
<213>  Mouse

<400>  50
gtgggacgca atattggc                                                    18


<210>  51
<211>  20
<212>  DNA
<213>  Mouse

<400>  51
tcccacattt ctggcataca                                                  20


<210>  52
<211>  21
<212>  DNA
<213>  Mouse

<400>  52
cacatgtgca tttaagcatg c                                                21


<210>  53
<211>  18
<212>  DNA
<213>  Mouse

<400>  53
gtgaggggtc aatgccac                                                    18


<210>  54
<211>  21
<212>  DNA
<213>  Mouse

<400>  54
ggctcagttg taagcacaag g                                                21


<210>  55
<211>  18
<212>  DNA
<213>  Mouse

<400>  55
cctaaccctc cccacacc                                                    18


<210>  56
<211>  23
<212>  DNA
<213>  Mouse

<400>  56
aaagatctgg attcaaatcc tcc                                              23


<210>  57
<211>  22
<212>  DNA
<213>  Mouse

<400>  57
acgagttgtc ctctgatcaa ca                                                22

<210>  58
<211>  20
<212>  DNA
<213>  Mouse

<400>  58
agccagagca aacaccaact                                                   20

<210>  59
<211>  21
<212>  DNA
<213>  Mouse

<400>  59
tatggatcca ctctccagaa a                                                 21

<210>  60
<211>  20
<212>  DNA
<213>  Mouse

<400>  60
caaagtctcc tccaaggctg                                                   20

<210>  61
<211>  19
<212>  DNA
<213>  Mouse

<400>  61
ctgctgcagc gattctctc                                                    19

<210>  62
<211>  22
<212>  DNA
<213>  Mouse

<400>  62
tcaggcacct aagtacatgt gc                                                22

<210>  63
<211>  26
<212>  DNA
<213>  Mouse

<400>  63
aaaaatcgtt ctttgacttc tacatg                                            26

<210>  64
<211>  25
<212>  DNA
<213>  Mouse

<400>  64
tttaaaaggg tttctttatc ctgtg                                             25

<210>  65

<211>  25
<212>  DNA
<213>  Mouse

<400>  65
ttttcttaga acataccttg cttgg                                          25


<210>  66
<211>  22
<212>  DNA
<213>  Mouse

<400>  66
ggggaaggtg aaaatttaaa gg                                             22


<210>  67
<211>  20
<212>  DNA
<213>  Mouse

<400>  67
tctccacgtg tgtgccttag                                                20


<210>  68
<211>  21
<212>  DNA
<213>  Mouse

<400>  68
tgaaagcact ctgcagactc a                                              21


<210>  69
<211>  20
<212>  DNA
<213>  Mouse

<400>  69
cactacacgg ggctgagatt                                                20


<210>  70
<211>  21
<212>  DNA
<213>  Mouse

<400>  70
   aagatccaac accctttttg g                                               21


<210>  71
<211>  20
<212>  DNA
<213>  Mouse

<400>  71
ggcagctttg tgctttaaaa                                                20


<210>  72
<211>  19
<212>  DNA
<213>  Mouse

<400>  72

atggatgttt acctggcgt                                                    19

<210>  73
<211>  26
<212>  DNA
<213>  Mouse

<400>  73
actggaaaat atgttttaaa cctctg                                            26

<210>  74
<211>  21
<212>  DNA
<213>  Mouse

<400>  74
aaatgggatt ctgcaaaaac c                                                 21

<210>  75
<211>  20
<212>  DNA
<213>  Mouse

<400>  75
gtgcactttc catgcctgta                                                   20

<210>  76
<211>  27
<212>  DNA
<213>  Mouse

<400>  76
gagtagaaag agacagagaa agacaca                                           27

<210>  77
<211>  20
<212>  DNA
<213>  Mouse

<400>  77
tctccagccc cttcattatg                                                   20

<210>  78
<211>  19
<212>  DNA
<213>  Mouse

<400>  78
tgccaaacac ccatgagac                                                    19

<210>  79
<211>  20
<212>  DNA
<213>  Mouse

<400>  79
tccttggtga gtccacatct                                                   20

<210>  80
<211>  19

<212>  DNA
<213>  Mouse

<400>  80
ggctaggctc tggaagaca                                                    19


<210>  81
<211>  23
<212>  DNA
<213>  Mouse

<400>  81
actagccata tggtttctga tgg                                               23


<210>  82
<211>  20
<212>  DNA
<213>  Mouse

<400>  82
gtagcagggc tgtgagcttt                                                   20


<210>  83
<211>  23
<212>  DNA
<213>  Mouse

<400>  83
tattaatgtt gaagccagaa gcc                                               23


<210>  84
<211>  23
<212>  DNA
<213>  Mouse

<400>  84
ctttaatcat ctctgtggca agg                                               23


<210>  85
<211>  23
<212>  DNA
<213>  Mouse

<400>  85
tgaagcaaga attcaagaag agg                                               23


<210>  86
<211>  25
<212>  DNA
<213>  Mouse

<400>  86
aggacatacc tagtaaggct ttgtc                                             25


<210>  87
<211>  23
<212>  DNA
<213>  Mouse

<400>  87
gatcttccta ccttcttacc cac                                               23

<210>  88
<211>  19
<212>  DNA
<213>  Mouse

<400>  88
catgatttta tttgggggg                                                    19


<210>  89
<211>  22
<212>  DNA
<213>  Mouse

<400>  89
gtcagacatt ggcttaggat cc                                                22


<210>  90
<211>  20
<212>  DNA
<213>  Mouse

<400>  90
ggtttgtgcg ctctctctct                                                   20


<210>  91
<211>  20
<212>  DNA
<213>  Mouse

<400>  91
tttgcctgta acaagccaga                                                   20


<210>  92
<211>  24
<212>  DNA
<213>  Mouse

<400>  92
ttgaggctat cagtttaaaa tccc                                              24


<210>  93
<211>  24
<212>  DNA
<213>  Mouse

<400>  93
gaataaccca tgtttatatc ggtg                                              24


<210>  94
<211>  22
<212>  DNA
<213>  Mouse

<400>  94
ctctggactt gtgttctatg cc                                                22


<210>  95
<211>  21
<212>  DNA

<213>  Mouse

<400>  95
cctcctaata gtgccattcc c                                                21


<210>  96
<211>  20
<212>  DNA
<213>  Mouse

<400>  96
ccagcaatca cgagtcttcc                                                  20


<210>  97
<211>  24
<212>  DNA
<213>  Mouse

<400>  97
gaccaaagtc atatctataa cacc                                             24


<210>  98
<211>  27
<212>  DNA
<213>  Mouse

<400>  98
ttaattgcaa gtaacacaat gagtagg                                          27


<210>  99
<211>  18
<212>  DNA
<213>  Mouse

<400>  99
actgttgctg gggaatgg                                                    18


<210>  100
<211>  19
<212>  DNA
<213>  Mouse

<400>  100
ccaagttcaa agctgctgg                                                   19


<210>  101
<211>  26
<212>  DNA
<213>  Mouse

<400>  101
agtccacctt aagaggacca agtagc                                           26


<210>  102
<211>  21
<212>  DNA
<213>  Mouse

<400>  102
tgtacagaaa caggacgcgg a                                                21

<210>  103
<211>  20
<212>  DNA
<213>  Mouse

<400>  103
tctccctcct tgctctgcag                                                    20


<210>  104
<211>  26
<212>  DNA
<213>  Mouse

<400>  104
tacctcgcag atgaccttag aattaa                                             26


<210>  105
<211>  28
<212>  DNA
<213>  Mouse

<400>  105
cctagtggaa agaatgtctc ctacaaca                                           28


<210>  106
<211>  26
<212>  DNA
<213>  Mouse

<400>  106
catattctgt gtggttgtta ggctca                                             26


<210>  107
<211>  24
<212>  DNA
<213>  Mouse

<400>  107
ccaggtacag tctttgatcc agga                                               24


<210>  108
<211>  22
<212>  DNA
<213>  Mouse

<400>  108
ccagggagtc tctgctggtc ta                                                 22


<210>  109
<211>  28
<212>  DNA
<213>  Mouse

<400>  109
catctgtgga agaactacac cagaagtc                                           28


<210>  110
<211>  22
<212>  DNA
<213>  Mouse

<400>  110
tacctcagag agctggccga gt                                                22

<210>  111
<211>  22
<212>  DNA
<213>  Mouse

<400>  111
ccaatgctga cctaatgttg gc                                                22

<210>  112
<211>  28
<212>  DNA
<213>  Mouse

<400>  112
catctataca cccgtgtgtg tatacaca                                          28

<210>  113
<211>  20
<212>  DNA
<213>  Mouse

<400>  113
aacttgtctt tgtccgggcc                                                   20

<210>  114
<211>  20
<212>  DNA
<213>  Mouse

<400>  114
ggcacagttc atcctcaccc                                                   20

<210>  115
<211>  25
<212>  DNA
<213>  Human

<400>  115
tagaatacag gctcatgttg caggt                                             25

<210>  116
<211>  23
<212>  DNA
<213>  Human

<400>  116
gccttcagca aatagcatga cgt                                               23

<210>  117
<211>  20
<212>  DNA
<213>  Mouse

<400>  117
gccactacca tccgattctt                                                   20

<210>  118
<211>  20
<212>  DNA
<213>  Mouse

<400>  118
cctgtcttga aaacccaaaa                                                   20


<210>  119
<211>  20
<212>  DNA
<213>  Mouse

<400>  119
aatgacttgt ttgccaagga                                                   20


<210>  120
<211>  20
<212>  DNA
<213>  Mouse

<400>  120
ttttatgccc taggagcctc                                                   20


<210>  121
<211>  21
<212>  DNA
<213>  Mouse

<400>  121
actctctgag tagcctgccc c                                                 21


<210>  122
<211>  20
<212>  DNA
<213>  Mouse

<400>  122
cctcaccctt acttggcacc                                                   20


<210>  123
<211>  11
<212>  DNA
<213>  Human

<400>  123
ctcgagccac c                                                            11


<210>  124
<211>  402
<212>  DNA
<213>  Human

<400>  124
atgtggcccc tggtagcggc gctgttgctg ggctcggcgt gctgcggatc agctcagcta      60

ctatttaata aaacaaaatc tgtagaattc acgttttgta atgacactgt cgtcattcca     120

tgctttgtta ctaatatgga ggcacaaaac actactgaag tatacgtaaa gtggaaattt     180

aaaggaagag atatttacac ctttgatgga gctctaaaca agtccactgt ccccactgac     240

tttagtagtg caaaaattga agtctcacaa ttactaaaag gagatgcctc tttgaagatg 300

gataagagtg atgctgtctc acacacagga aactacactt gtgaagtaac agaattaacc 360

agagaaggtg aaacgatcat cgagctaaaa tatcgtgttg tt 402

<210> 125
<211> 27
<212> DNA
<213> Human

<400> 125
acggatcccg agggtgagta ctaagct 27

<210> 126
<211> 1267
<212> DNA
<213> Human

<400> 126
tcagcgctcc tgcctggacg catcccggct atgcagcccc agtccagggc agcaaggcag 60

gccccgtctg cctcttcacc cggagcctct gcccgcccca ctcatgctca gggagagggt 120

cttctggctt tttcccaggc tctgggcagg cacaggctag gtgcccctaa cccaggccct 180

gcacacaaag gggcaggtgc tgggctcaga cctgccaaga gccatatccg ggaggaccct 240

gcccctgacc taagcccacc ccaaaggcca aactctccac tccctcagct cggacacctt 300

ctctcctccc agattccagt aactcccaat cttctctctg cagagcccaa atcttgtgac 360

aaaactcaca catgcccacc gtgcccaggt aagccagccc aggcctcgcc ctccagctca 420

aggcgggaca ggtgccctag agtagcctgc atccagggac aggccccagc cgggtgctga 480

cacgtccacc tccatctctt cctcagcacc tgaactcctg gggggaccgt cagtcttcct 540

cttcccccca aaacccaagg acaccctcat gatctcccgg acccctgagg tcacatgcgt 600

ggtggtggac gtgagccacg aagaccctga ggtcaagttc aactggtacg tggacggcgt 660

ggaggtgcat aatgccaaga caaagccgcg ggaggagcag tacaacagca cgtaccgggt 720

ggtcagcgtc ctcaccgtcc tgcaccagga ctggctgaat ggcaaggagt acaagtgcaa 780

ggtctccaac aaagccctcc cagcccccat cgagaaaacc atctccaaag ccaaaggtgg 840

gacccgtggg gtgcgagggc cacatggaca gaggccggct cggcccaccc tctgccctga 900

gagtgaccgc tgtaccaacc tctgtcctac agggcagccc cgagaaccac aggtgtacac 960

cctgccccca tcccgggatg agctgaccaa gaaccaggtc agcctgacct gcctggtcaa 1020

aggcttctat cccagcgaca tcgccgtgga gtgggagagc aatgggcagc cggagaacaa 1080

ctacaagacc acgcctcccg tgctggactc cgacggctcc ttcttcctct acagcaagct 1140

caccgtggac aagagcaggt ggcagcaggg gaacgtcttc tcatgctccg tgatgcatga 1200

ggctctgcac aaccactaca cgcagaagag cctctccctg tctccgggta aatgagtgcg 1260

acggccg 1267